(19) Europäisches Patentamt
European Patent Office
Office européen des brevets

(11) EP 0 474 846 B1

(12) **FASCICULE DE BREVET EUROPEEN**

(45) Date de publication et mention de la délivrance du brevet:
**23.07.1997 Bulletin 1997/30**

(21) Numéro de dépôt: **91907472.4**

(22) Date de dépôt: **03.04.1991**

(51) Int Cl.[6]: **C07K 14/00**, C12P 21/08, G01N 33/566, G01N 33/68, C12Q 1/68, C12P 19/34, C12N 15/12, C12N 1/21, C12N 5/10, A61K 38/00 // C12R1:19

(86) Numéro de dépôt international:
**PCT/FR91/00269**

**WO 91/15513 (17.10.1991 Gazette 1991/24)**

(54) **POLYPEPTIDES AYANT UNE ACTIVITE DE RECEPTEUR DOPAMINERGIQUE, ACIDES NUCLEIQUES CODANT POUR CES POLYPEPTIDES ET UTILISATION DE CES POLYPEPTIDES POUR LE CRIBLAGE DE SUBSTANCES ACTIVES SUR CES POLYPEPTIDES**

POLYPEPTIDE MIT DOPAMINERGER REZEPTOR-AKTIVITÄT, FÜR SIE KODIERENDE NUKLEINSÄUREN UND IHRE VERWENDUNG

POLYPEPTIDES WITH A DOPAMINERGIC RECEPTOR ACTIVITY, NUCLEIC ACIDS CODING FOR THESE POLYPEPTIDES AND USE OF THESE POLYPEPTIDES TO SCREEN ACTIVE SUBSTANCES ON THESE POLYPEPTIDES

(84) Etats contractants désignés:
**AT BE CH DE DK ES FR GB GR IT LI LU NL SE**

(30) Priorité: **06.04.1990 FR 9004476**
**26.06.1990 FR 9008027**

(43) Date de publication de la demande:
**18.03.1992 Bulletin 1992/12**

(60) Demande divisionnaire: **97100517.8**

(73) Titulaire: **INSTITUT NATIONAL DE LA SANTE ET DE LA RECHERCHE MEDICALE (INSERM)**
**75654 Paris Cédex 13 (FR)**

(72) Inventeurs:
- **SOKOLOFF, Pierre**
  **F-95130 Le Plessis-Bouchard (FR)**
- **MARTRES, Marie-Pascale**
  **F-75015 Paris (FR)**
- **SCHWARTZ, Jean-Charles**
  **F-75014 Paris (FR)**
- **GIROS, Bruno**
  **F-92320 Châtillon (FR)**

(74) Mandataire: **Gutmann, Ernest et al**
**Ernest Gutmann - Yves Plasseraud S.A.**
**3, rue Chauveau-Lagarde**
**75008 Paris (FR)**

(56) Documents cités:
- **Molecular Pharmacology, vol. 21, 1982, The American Society for Pharmacology and Experimental Therapeutics, M.W. Hamblin et al.: "Phenoxybenzamine treatment differentiates dopaminergic 3H-ligand binding sites in bovine caudate membranes", pages 44-51**
- **Naunyn-Schmiedeberg's Arch. Pharmacol., vol. 315, 1980, Springer-Verlag, P. Sokoloff et al.: "Three classes of dopamine receptor (D-2, D-3, D-4) identified by binding studies with 3H-Apomorphine and 3H-domperidone", pages 89-102**
- **Nature, vol. 347, 13 septembre 1990, P. Sokoloff et al.: "Molecular cloning and characterization of a novel dopamine receptor (D3) as a target for neuroleptics", pages 146-151**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 474 846 B1

Printed by Jouve, 75001 PARIS (FR)

## Description

L'invention a pour objet des polypeptides ayant une activité de récepteur dopaminergique et les gènes codant pour ces polypeptides.

L'invention est également relative

- à des vecteurs contenant les gènes codant pour des polypeptides ayant une activité de récepteur dopaminergique,
- à des cellules transformées pour exprimer les gènes susdits sur lesquelles il est possible d'évaluer l'activité d'agents agonistes ou antagonistes desdits récepteurs,
- à des sondes nucléotidiques susceptibles de s'hybrider avec les gènes codant pour les susdits polypeptides, lesquelles sondes seraient susceptibles d'être utilisées pour le diagnostic in vitro d'affections, notamment neurologiques, psychiatriques, cardio-vasculaires ou neuroendocriniennes,
- à des anticorps polyclonaux et monoclonaux dirigés contre les susdits polypeptides et utilisables dans un but analytique pour purifier lesdits polypeptides, de diagnostic in vitro, ou dans un but thérapeutique, notamment dans les affections impliquant les systèmes dopaminergiques,
- à des trousses (ou kits) pour étudier le degré d'affinité de certaines substances pour les susdits polypeptides ,
- à des médicaments destinés plus particulièrement au traitement des affections psychiatriques, neurologiques, cardio-vasculaires ou neuroendocriniennes et contenant des substances actives sur les susdits polypeptides ayant une activité de récepteur dopaminergique, ou contenant des substances actives sur des cellules transfectées par les gènes ou fragments de gènes codant pour lesdits polypeptides à activité de récepteur dopaminergique,
- à des médicaments actifs sur les récepteurs dopaminergiques déjà identifiés mais dont on voudrait diminuer certains effets secondaires attribuables à leur interaction avec lesdits polypeptides.

Il était admis jusqu'alors que les divers effets de la dopamine résultaient de son interaction avec deux types de récepteurs communément désignés sous les noms de récepteurs D-1 et D-2 (Kekabian J.W. et Calne D.B., "Multiple receptors for dopamine". Nature, 1979, 277:93-96). Parmi ceux-ci, seule la séquence du récepteur D-2 était connue (Bunzow J.R., Van Tol H.H.M., Grandy D.K., Albert P., Salon J., Christie Mc D., Machida C.A., Neve K.A. et Civelli O. "Cloning and expression of a D-2 dopamine receptor cDNA". Nature, 1988, 336: 783-787) et deux isoformes du récepteur D-2 (parfois désignées D-2A et D-2B ou encore D-2(415) et D-2(444) résultant de l'épissage alternatif de l'ARN messager du gène dudit récepteur avaient été décrites (Giros B., Sokoloff P., Martres M.P., Riou J.F., Emorine L.J. et Schwartz J.C. "Alternative splicing directs the expression of two D-2 dopamine receptor isoforms". Nature, 1989, 342: 923-926 ; Dal Toso R., Sommer B., Ewert M., Herb A., Pritchett D.B., Bach A., Shivers B.D. et Seeburg P.H. "The dopamine D-2 receptor : two molecular forms generated by alternative splicing". The EMBO Journal, 1989, 8: 4025-4034).

Le récepteur D-2 appartient à la famille des récepteurs comportant sept domaines transmembranaires, couplés à une protéine G et présentant un certain degré d'homologie avec les récepteurs de la famille des rhodopsines qui sont exprimés par les photorécepteurs de la rétine.

L'existence de récepteurs dopaminergiques distincts des récepteurs D-1 et D-2 avait été suspectée par divers auteurs sur des observations indirectes (voir notamment Schwartz J.C., Delandre M., Martres M.P., Sokoloff P., Protais P., Vasse M., Costentin J., Laibe P., Wermuth C.G., Gulat C. et Lafitte A. "Biochemical and behavioral identification of discriminant benzamide derivatives : new tools to differentiate subclasses of dopamine receptors". Catecholamines: neuropharmacology and central nervous system. Theoretical aspects. Alan R. Liss, Inc. N.Y. 1984, pp. 59-72) mais n'avait jamais été prouvée car lesdits récepteurs n'avaient jamais été isolés, ni identifiés quant à leur structure, ni entièrement définis par leurs propriétés pharmacologiques. En particulier, l'existence d'autorécepteurs régulant la synthèse et/ou la libération de dopamine et/ou encore l'activité des neurones dopaminergiques avait été démontrée mais il était couramment admis qu'ils étaient identiques aux récepteurs D-2 en ce qui concerne leur pharmacologie.

L'invention a pour objet de donner accès à de nouveaux polypeptides ayant une activité de récepteur dopaminergique, ne s'apparentant ni à celle des récepteurs dopaminergiques D-1, ni à celle des récepteurs dopaminergiques D-2.

L'invention a également pour objet des procédés de criblage de nouveaux médicaments agissant sur les nouveaux polypeptides ayant une action de récepteur dopaminergique ou dont on voudrait au contraire éviter l'interaction avec les nouveaux polypeptides, et destinés, entre autres, au traitement des troubles psychiatriques, neurologiques, cardio-vasculaires ou neuroendocriniens.

En particulier, le nouveau polypeptide de l'invention ayant une activité de récepteur dopaminergique :

- contient la séquence de 446 acides aminés de la Figure 1 ou un fragment de cette séquence, ce fragment étant tel que

  * soit il contient néanmoins les sites contenus dans cette séquence et dont la présence est nécessaire pour que, lorsque ce fragment est présent dans une membrane, il soit capable de lier la dopamine à ses agonistes ou antagonistes de manière spécifique et mesurable, par exemple au moyen d'un ligand radioactif selon la technique décrite dans Sokoloff P., Martres M.P. et Schwartz J.C. "Three classes of dopamine receptor (D-2, D-3, D-4) identified by binding studies with $^3$H-apomorphine and $^3$H-domperidone". Naunyn-Schmiedeberg's Arch. Pharmacol. 1980, 315: 89-102 et dans Martres M.P., Bouthenet M.L., Sales N., Sokoloff P. et Schwartz J.C. "Widespread distribution of brain dopamine receptors evidenced with $^{125}$I-iodosulpride, a highly selective ligand". Science, 1985, 228: 752-755.

* soit il est susceptible d'être reconnu par des anticorps qui reconnaissent également la susdite séquence de 446 acides aminés, mais ne reconnaissent ni le récepteur dopaminergique D-1, ni le récepteur dopaminergique D-2,
* soit il est susceptible de générer des anticorps qui reconnaissent la susdite séquence de 446 acides aminés mais ne reconnaissent ni le récepteur dopaminergique D-1, ni le récepteur dopaminergique D-2,

ou les polypeptides variants qui correspondent aux polypeptides sus-définis, comportant certaines mutations localisées, sans que les polypeptides ne perdent les propriétés de récepteur dopaminergique.

En particulier, un polypeptide variant avantageux de l'invention ayant une activité de récepteur dopaminergique :

- contient la séquence de 446 acides aminés de la Figure 2 ou un fragment de cette séquence, ce fragment étant tel que

  * soit il contient néanmoins les sites contenus dans cette séquence et dont la présence est nécessaire pour que, lorsque ce fragment est présent dans une membrane, il soit capable de lier la dopamine à ses agonistes ou antagonistes de manière spécifique et mesurable, par exemple au moyen d'un ligand radioactif selon la technique décrite dans Martres, M.P., Bouthenet, M.L., Salés, N., Sokoloff, P. et Schwartz, J.C. Science 228, 752-755 (1985),
  * soit il est susceptible d'être reconnu par des anticorps qui reconnaissent également la susdite séquence de 446 acides aminés, mais ne reconnaissent ni le récepteur dopaminergique D-1, ni le récepteur dopaminergique D-2,
  * soit il est susceptible de générer des anticorps qui reconnaissent la susdite séquence de 446 acides aminés mais ne reconnaissent ni le récepteur dopaminergique D-1, ni le récepteur dopaminergique D-2.

La reconnaissance de l'une des susdites séquences de 446 acides aminés par les susdits anticorps - ou du susdit fragment par les susdits anticorps - signifie que la susdite séquence forme un complexe avec l'un des susdits anticorps.

La formation du complexe antigène (c'est-à-dire séquence de 446 acides aminés ou susdit fragment)- anticorps et la détection de l'existence d'un complexe formé peuvent se faire par des techniques classiques (telles que celles utilisant un traceur marqué par des isotopes radioactifs ou une enzyme).

Pour ce qui est de la définition des "fragments contenant les sites" et répondant à la susdite définition, on se reportera ci-après dans la description.

Parmi les fragments ci-dessus définis, on préfère le ou les fragments produits par épissage alternatif de l'ARN messager produit par le gène codant pour l'une des deux séquences de 446 acides aminés définie ci-dessus.

De façon avantageuse, les polypeptides de l'invention correspondent à des fragments de la séquence représentée sur la Figure 2, qui contiennent l'acide aminé correspondant à celui en position 139.

Des polypeptides avantageux de l'invention sont tels qu'ils contiennent les sites contenus dans l'une des susdites séquences de 446 acides aminés, dont la présence est nécessaire pour que, lorsque ces polypeptides sont exposés à la surface d'une cellule en présence d'iodosulpride $^{125}$I, et en présence d'un agoniste, l'affinité respective des agonistes suivants: quinpirole, apomorphine, dopamine, vis-à-vis des polypeptides de l'invention est dans l'ordre suivant :

- l'affinité du quinpirole vis-à-vis des polypeptides de l'invention est supérieure à l'affinité de l'apomorphine vis-à-vis des polypeptides de l'invention,
- l'affinité de l'apomorphine vis-à-vis des polypeptides de l'invention est sensiblement égale à l'affinité de la dopamine vis-à-vis des polypeptides de l'invention.

Des peptides avantageux de l'invention sont tels qu'ils contiennent les sites contenus dans l'une des susdites séquences de 446 acides aminés, dont la présence est nécessaire pour que, lorsque ces polypeptides sont exposés à la surface d'une cellule en présence d'iodosulpride $^{125}$I, et en présence d'un antagoniste , l'affinité respective des antagonistes suivants : raclopride, UH 232, halopéridol vis-à-vis des polypeptides de l'invention est dans l'ordre suivant :

- l'affinité du raclopride vis-à-vis des peptides de l'invention est supérieure à l'affinité de UH 232 vis-à-vis des polypeptides de l'invention, et
- l'affinité de UH 232 vis-à-vis des polypeptides de l'invention est sensiblement égale à l'affinité de l'halopéridol vis-à-vis des polypeptides de l'invention.

UH 232 est défini dans l'article de Svensson K. et al. "(+)- AJ 76 and (+)-UH 232 : central stimulants acting as preferential dopamine autoreceptor antagonists". Naunyn-Schmiedeberg's Arch. Pharmacol., 1986, 334, 234-245.

Dans ce qui précède et dans ce qui suit, un ligand est considéré comme "agoniste" ou "antagoniste", par référence au comportement connu de ce ligand vis-à-vis du récepteur D-2.

La mesure de l'affinité d'un agoniste ou d'un antagoniste vis-à-vis des polypeptides de l'invention se fait par compétition entre l'agoniste ou l'antagoniste (dont on veut mesurer l'affinité) et l'iodosulpride $^{125}I$, qui est un ligand à haute affinité des polypeptides de l'invention.

En ce qui concerne la mesure de l'affinité des agonistes ou des antagonistes vis-à-vis des polypeptides de l'invention, elle peut avoir lieu dans les conditions suivantes :

on utilise une solution (volume final = 400µl) de tampon Tris-HCl 50mM, contenant NaCl 120mM, KCl 5mM, $CaCl_2$ 2mM, $MgCl_2$ 2mM, 0,1% d'acide ascorbique, 8-hydroxy quinoléine 10µM, 0,1% d'albumine de sérum bovin et iodosulpride-$^{125}I$ 0,1 à 0,2nM , 5 µg de protéines de membrane provenant de cellules CHO transfectées, dans lesquelles les polypeptides sont présents à raison d'environ lpmol/mg de protéine membranaire.

L'invention concerne également des protéines chimères dans lesquelles le polypeptide tel que défini plus haut ou des parties de celui-ci sont réunis à un enchaînement d'acides aminés hétérologue par rapport à ce polypeptide.

Les polypeptides et protéines chimères de l'invention peuvent être glycosylés et peuvent comporter ou non des ponts disulfure.

Dans la suite de la description, les polypeptides de l'invention seront, pour simplifier, désignés par "récepteurs D-3".

Un récepteur D-3 avantageux de l'invention est constitué par l'enchaînement des acides aminés 1 à 446, représentés sur la Figure 1 ou par l'enchaînement des acides aminés 1 à 446 représenté sur la Figure 2.

Ce récepteur D-3 est considéré comme comportant sept régions transmembranaires hydrophobes séparées par des boucles hydrophiles intra et extracellulaires.

L'invention concerne également les polypeptides variants qui correspondent aux polypeptides sus-définis comportant certaines mutations localisées, sans que les polypeptides ne perdent les propriétés de récepteur D-3 dopaminergique, et qui sont reconnus par des anticorps reconnaissant les régions transmembranaires, ainsi que ceux qui sont reconnus par des anticorps reconnaissant les régions autres que les régions transmembranaires.

L'invention concerne également des acides nucléiques qui comprennent ou qui sont constitués par un enchaînement de nucléotides codant pour l'un quelconque des récepteurs D-3 précédemment définis.

Plus particulièrement, l'invention concerne l'acide nucléique qui comprend l'enchaînement de nucléotides représenté sur la Figure 3, s'étendant de l'extrémité constituée par le nucléotide en position 1 à celle constituée par le nucléotide en position 1863.

Plus particulièrement, l'invention concerne également l'acide nucléique qui comprend l'enchaînement de nucléotides représenté sur la Figure 4, s'étendant de l'extrémité constituée par le nucléotide en position 1 à celle constituée par le nucléotide en position 1863.

L'invention concerne particulièrement l'acide nucléique représenté sur la Figure 2 comprenant ou étant constitué par l'enchaînement s'étendant de l'extrémité constituée par le nucléotide en position 442 à celle constituée par le nucléotide en position 1779.

L'acide nucléique sus-défini correspond à la partie codante du gène correspondant au polypeptide représenté sur la Figure 1.

L'invention concerne particulièrement l'acide nucléique représenté sur la Figure 4 comprenant ou étant constitué par l'enchaînement s'étendant de l'extrémité constituée par le nucléotide en position 442 à celle constituée par le nucléotide en position 1779.

L'acide nucléique sus-défini correspond à la partie codante du gène correspondant au polypeptide représenté sur la Figure 2.

De préférence, les acides nucléiques de l'invention sont tels qu'ils correspondent à des fragments de l'enchaînement de nucléotides représenté sur la Figure 4 et contiennent les nucléotides correspondant respectivement à ceux situés aux positions 856, 857 et 859.

Font également partie de l'invention les acides nucléiques variants par rapport à ceux sus-définis et qui comportent certaines mutations localisées dans la mesure où ces acides nucléiques variants s'hybrident avec les acides nucléiques précédemment définis ou avec les sondes nucléiques définies ci-après dans les conditions d'hybridation définies ci-après dans la description.

Les acides nucléiques de l'invention peuvent être préparés soit par un procédé chimique, soit par d'autres procédés.

Un mode de préparation approprié des acides nucléiques (comportant au maximum 200 nucléotides - ou pb, lorsqu'il s'agit d'acides nucléiques bicaténaires) de l'invention par voie chimique comprend les étapes suivantes :

- la synthèse d'ADN en utilisant la méthode automatisée de β-cyanéthyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides - ou pb (lorsqu'il s'agit d'acides nucléiques bicaténaires) comprend les étapes suivantes:

- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

Un autre procédé de préparation des acides nucléiques de l'invention à partir d'ARNm comprend les étapes suivantes :

- préparation d'ARN cellulaire à partir de tout tissu exprimant le récepteur dopaminergique D-3 selon les techniques décrites par Maniatis et al., "Molecular cloning", Cold Spring Harbor Laboratory, 1982, et Ausubel F.M., Brent R., Kingston R.E., Moore D.D., Smith J.A., Seidman J.G. et Struhl K. (1989) Current Protocols in Molecular Biology, chapitre 4, Green Publishing Associates et Wiley-Interscience, New York,
- récupération et purification des ARNm par passage des ARN cellulaires totaux par chromatographie avec un oligodT immobilisé,
- synthèse d'un brin d'ADNc à partir des ARNm purifiés d'après la technique décrite dans Gene 25:263, 1983,
- clonage des acides nucléiques ainsi obtenus dans un vecteur plasmidien approprié et récupération de la séquence nucléotidique recherchée en utilisant une sonde d'hybridation appropriée.

Pour préparer les acides nucléiques de l'invention, les sondes d'hybridation oligonucléotidiques synthétisées chimiquement peuvent être les suivantes :

. - celle définie par la séquence d'acides nucléiques représentée sur la Figure 3, s'étendant de l'extrémité constituée par le nucléotide en position 1, à celle constituée par le nucléotide en position 441,
. - celle définie par la séquence d'acides nucléiques représentée sur la Figure 3, s'étendant de l'extrémité constituée par le nucléotide en position 442, à celle constituée par le nucléotide en position 537,
. - celle définie par la séquence d'acides nucléiques représentée sur la Figure 3, s'étendant de l'extrémité constituée par le nucléotide en position 718, à celle constituée par le nucléotide en position 753,
. - celle définie par la séquence d'acides nucléiques représentée sur la Figure 3, s'étendant de l'extrémité constituée par le nucléotide en position 820, à celle constituée par le nucléotide en position 888,
. - celle définie par la séquence d'acides nucléiques représentée sur la Figure 3, s'étendant de l'extrémité constituée par le nucléotide en position 958, à celle constituée par le nucléotide en position 996,
. - celle définie par la séquence d'acides nucléiques représentée sur la Figure 3, s'étendant de l'extrémité constituée par le nucléotide en position 1068, à celle constituée par le nucléotide en position 1240,
. - celle définie par la séquence d'acides nucléiques représentée sur la Figure 3, s'étendant de l'extrémité constituée par le nucléotide en position 1068, à celle constituée par le nucléotide en position 1566,

ou leur séquence nucléotidique complémentaire;
ce sont des séquences qui ont été dérivées de celles de la Figure 3 et elles peuvent être utilisées dans les conditions d'hybridation décrites par Maniatis et al., "Molecular cloning", Cold Spring Harbor Laboratory, 1982.

Pour préparer les acides nucléiques de l'invention, une autre sonde d'hybridation oligonucléotidique synthétisée chimiquement peut être la suivante :

. - celle définie par la séquence d'acides nucléiques représentée sur la Figure 4, s'étendant de l'extrémité constituée par le nucléotide en position 820, à celle constituée par le nucléotide en position 888

cette séquence dérive des séquences de la Figure 4 et elle peut être utilisée dans les conditions d'hybridation décrites par Maniatis et al. (1982) déjà cité.

La synthèse du brin d'ADNc et son amplification subséquente in vitro peut également être effectuée en utilisant la méthode PCR (Polymerase Chain Reaction), comme décrit par exemple par Goblet et al. Nucleic Acid Research, 17, 2144, 1989 "One step amplification of transcripts in total RNA using Polymerase Chain Reaction", en utilisant deux amplimères chimiquement synthétisés définis à partir de la séquence de la Figure 3. Des amplimères appropriés sont par exemple : celui défini sur la Figure 3 du nucléotide 410 au nucléotide 433 et celui défini sur la Figure 3 du nucléotide 1800 au nucléotide 1825.

Le fragment d'acides nucléiques amplifié peut être ensuite cloné selon les techniques décrites dans Ausubel F. M., Brent R., Kingston R.E., Moore D.D., Smith J.A., Seidman J.G. et Struhl K. (1989) Current Protocols in Molecular Biology, chapitre 3, Green Publishing Associates et Wiley-Interscience, New York.

L'invention concerne également les vecteurs recombinants, en particulier pour le clonage et/ou l'expression, notamment du type plasmide, cosmide, phage, ou virus, contenant un acide nucléique de l'invention en l'un de ses sites non essentiels pour sa réplication.

Un vecteur approprié de l'invention, contient en l'un de ses sites non essentiels pour sa réplication des éléments nécessaires pour promouvoir l'expression d'un polypeptide selon l'invention, dans un hôte cellulaire et éventuellement un promoteur reconnu par les polymérases de l'hôte cellulaire, en particulier un promoteur inductible et éventuellement une séquence signal et une séquence d'ancrage.

L'invention concerne également un hôte cellulaire transformé par un vecteur recombinant défini précédemment comprenant les éléments de régulation permettant l'expression de la séquence nucléotidique codant pour l'un des polypeptides selon l'invention dans cet hôte.

Par hôte cellulaire on entend tout organisme susceptible d'être maintenu en culture.

L'un des microorganismes utilisés peut être constitué par une bactérie, notamment Escherichia coli.

Un organisme de choix est constitué par un organisme eucaryote tel que des cellules CHO (Chinese Hamster Ovary) ou COS-7 (fibroblaste de rein de singe vert africain transformé par le virus SV-40).

Mais d'autres organismes peuvent être utilisés tout aussi aisément, naturellement sous réserve que l'on dispose pour chacun d'entre eux des vecteurs, notamment plasmidiques, susceptibles de s'y répliquer et des séquences de nucléotides insérables dans ces vecteurs et capables, lorsqu'elles sont suivies dans ces vecteurs par un insérat codant pour un polypeptide de l'invention, d'assurer l'expression de cet insérat dans les organismes choisis et leur transport dans les membranes de ces hôtes cellulaires.

L'invention concerne également les anticorps dirigés de façon spécifique contre l'un des polypeptides de l'invention, ces anticorps étant tels qu'ils ne reconnaissent ni le récepteur dopaminergique D-1, ni le récepteur dopaminergique D-2. En particulier, ces anticorps reconnaissent les séquences d'acides aminés suivantes :

- celle définie par la séquence d'acides aminés représentée sur la Figure 1 s'étendant de l'extrémité constituée par l'acide aminé en position 1 à celle constituée par l'acide aminé en position 38,
- celle définie par la séquence d'acides aminés représentée sur la Figure 1 s'étendant de l'extrémité constituée par l'acide aminé en position 135 à celle constituée par l'acide aminé en position 147,
- celle définie par la séquence d'acides aminés représentée sur la Figure 1 s'étendant de l'extrémité constituée par l'acide aminé en position 175 à celle constituée par l'acide aminé en position 187,
- celle définie par la séquence d'acides aminés représentée sur la Figure 1 s'étendant de l'extrémité constituée par l'acide aminé en position 210 à celle constituée par l'acide aminé en position 375,
- celle définie par la séquence d'acides aminés représentée sur la Figure 2 s'étendant de l'extrémité constituée par l'acide aminé en position 135 à celle constituée par l'acide aminé en position 147.

Pour obtenir les anticorps, on peut injecter chez l'animal l'un des susdits polypeptides.

On prépare des anticorps monoclonaux par fusion cellulaire entre des cellules de myélome et des cellules spléniques de souris immunisées, selon les procédés classiques.

Les anticorps de l'invention peuvent être utilisés pour le diagnostic in vitro du caractère tumoral ou non de certaines cellules ainsi que du caractère bénin ou malin de certaines tumeurs, dans la mesure où ces éléments sont corrélés à l'expression pathologique du récepteur D-3.

On a pu constater en effet, que dans certaines tumeurs, notamment tumeurs du poumon, il est possible de détecter l'expression de récepteurs dopaminergiques qui ne devraient pas être normalement présents (Sokoloff P., Riou J.F., Martres M.P. et Schwartz J.C. "Presence of dopamine D-2 receptors in human tumoral cell lines". Biochem. Biophys. Res. Comm. 1989, 162: 575-582).

Ce procédé de diagnostic in vitro à partir du prélèvement biologique susceptible de contenir le récepteur dopaminergique D-3 comprend :

- la mise en contact d'un anticorps de l'invention avec le susdit prélèvement biologique dans des conditions permettant la production éventuelle d'un complexe immunologique formé entre le récepteur dopaminergique D-3 ou produit qui en a dérivé et l'anticorps de l'invention,
- la détection du susdit complexe immunologique formé.

L'invention concerne également les sondes nucléotidiques synthétiques ou non, s'hybridant avec l'un des acides nucléiques définis ci-dessus ou leurs séquences complémentaires ou leur ARN correspondant, ces sondes étant telles qu'elles s'hybrident ni avec le gène ni avec les ARN messagers des récepteurs D-1 et D-2 dopaminergiques.

Les sondes de l'invention comportent au minimum 10, avantageusement 15 acides nucléiques et peuvent comporter au maximum la totalité de la séquence nucléotidique représentée sur la Figure 3 ou sur la Figure 4.

Pour les sondes les plus courtes, c'est-à-dire d'environ 10 à environ 100 nucléotides, des conditions d'hybridation appropriées sont les suivantes:
900 mM de NaCl, 90 mM de tri-sodium citrate pH 7,0, 100 µg/ml d'ADN de sperme de saumon, 0,05% de pyrophosphate de sodium, 10 à 25% de formamide déionisée, 0,02% de Ficoll (Pm de 400.000), 0,02% d'albumine de sérum bovine, 0,02% de polyvinylpyrrolidone, pendant 14 à 16 heures à 42°C.

Pour les sondes les plus longues, c'est-à-dire présentant plus d'environ 100 nucléotides, des conditions d'hybridation appropriées sont les suivantes :
600 mM de NaCl, 60 mM de tri-sodium citrate, 20 µg/ml d'ADN de sperme de saumon, 20 µg/ml d'ARNt de levure, 8 mM de Tris-HCl pH 7,4, 40 à 60% de formamide déionisée, 0,02% de Ficoll, 0,02% d'albumine de sérum bovine, 0,02% de polyvinylpyrrolidone, 0,2% de sodium dodécyl sulfate, 10% de sulfate de dextrane.

L'invention concerne en particulier les sondes nucléotidiques définies précédemment.

Les sondes de l'invention peuvent être utilisées comme outils de diagnostic notamment in vitro d'affections neurologiques, psychiatriques et cardio-vasculaires.

Les sondes de l'invention peuvent également servir à détailler l'épissage alternatif de l'ARN messager produit par le gène codant pour la séquence de 446 acides aminés définie ci-dessus, l'existence ou non de cet épissage pouvant être liée à des pathologies neurologiques, psychiatriques, cardio-vasculaires ou neuroendocriniennes.

Plus particulièrement, les sondes de l'invention peuvent être utilisées pour détecter les anomalies génétiques, notamment polymorphismes ou mutations ponctuelles.

En ce qui concerne la détection des polymorphismes du gène codant pour le récepteur dopaminergique D-3 chez un individu, elle peut être réalisée à partir d'un échantillon biologique tel que le sang, prélevé chez l'individu selon un procédé comprenant les étapes suivantes :

- le traitement de l'acide nucléique issu de l'échantillon biologique sus-mentionné réalisé à l'aide d'une enzyme de restriction dans des conditions permettant l'obtention de fragments de restriction issus du clivage dudit acide nucléique au niveau de ces sites de restriction reconnus par ladite enzyme,
- la mise en contact d'une sonde nucléotidique de l'invention, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

En ce qui concerne la méthode de diagnostic in vitro de détection des mutations ponctuelles de l'acide nucléique ou d'un fragment de l'acide nucléique codant pour le récepteur dopaminergique D-3 chez un individu, elle peut être effectuée selon le procédé comprenant les étapes suivantes :

- la mise en contact d'une sonde nucléotidique de l'invention avec un prélèvement biologique, par exemple du sang, dans des conditions permettant le production éventuelle d'un complexe d'hybridation formé entre la sonde et l'acide nucléique ou un fragment d'acide nucléique sus-mentionné,
- la détection du susdit complexe d'hybridation,
- le séquençage de l'acide nucléique ou du fragment d'acide nucléique intervenant dans le susdit complexe d'hybridation,
- la comparaison de la séquence de l'acide nucléique ou du fragment d'acide nucléique intervenant dans le susdit complexe d'hybridation avec la séquence de l'acide nucléique (ne présentant pas de mutation) ou du fragment de l'acide nucléique (ne présentant pas de mutation) du récepteur dopaminergique D-3.

En ce qui concerne la détection de l'épissage alternatif de l'ARN messager produit par le gène codant pour le récepteur D-3, elle peut se faire par quantification de l'ARN messager contenu dans un échantillon de tissu, en utilisant l'une des sondes de l'invention.

Les sondes de l'invention peuvent également être utilisées pour détecter l'expression pathologique du récepteur dopaminergique D-3, cette expression étant révélée par la présence d'ARN messager du récepteur dopaminergique D-3 dans des cellules où il ne devrait pas être présent.

Cette détection in vitro peut être effectuée à partir d'un échantillon biologique prélevé chez un individu, tel que le sang, selon un procédé qui comprend les étapes suivantes :

- l'amplification préalable possible des quantités de séquence nucléotidique susceptible d'être contenue dans un échantillon biologique prélevé sur un patient, au moyen d'une amorce d'ADN,
- la mise en contact de l'échantillon biologique indiqué ci-dessus avec une sonde nucléotidique, dans des conditions permettant la production d'un complexe d'hybridation formé de ladite sonde et ladite séquence nucléotidique,
- la détection du complexe d'hybridation ci-dessus qui a pu se former.

Cette expression pathologique du récepteur dopaminergique D-3 peut se manifester dans le cas de certaines tumeurs, et peut aussi être corrélée au caractère malin ou bénin de certaines tumeurs.

Les polypeptides de l'invention peuvent être préparés par culture dans un milieu approprié d'un hôte cellulaire préalablement transformé par un vecteur recombinant contenant l'un des acides nucléiques définis précédemment et par récupération à partir de la susdite culture du polypeptide produit par ledit hôte cellulaire transformé.

Un autre procédé de préparation des polypeptides de l'invention est caractérisé en ce que, partant de préférence de l'amino acide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amines de l'un et carboxyles de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

S'agissant de réaliser l'expression des récepteurs dopaminergiques D-3 dans une bactérie, telle que E. coli ou dans une cellule eucaryote telle qu'une cellule CHO, on effectue les étapes suivantes :

- la transformation d'un hôte cellulaire compétent avec un vecteur, notamment un plasmide ou un phage, dans lequel a auparavant été insérée une séquence de nucléotides codant pour le récepteur D-3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 exprimé vers la membrane de façon telle que les séquences transmembranaires du récepteur D-3 soient exposées à la surface de l'hôte cellulaire transformé.

S'agissant de l'expression en cellules eucaryotes, les éléments de régulation peuvent comporter le promoteur endogène des récepteurs dopaminergiques ou des promoteurs viraux tels que ceux des virus SV40 ou du virus du sarcome de Rous (RSV).

S'agissant de l'expression dans E. coli, les éléments de régulation peuvent comporter le promoteur de l'opéron lactose ou de l'opéron tryptophane.

L'invention concerne également un procédé de détection de la capacité d'une molécule dopaminergique à se comporter comme ligand vis-à-vis d'un polypeptide de l'invention, lequel procédé comprend :

- la mise en contact de la molécule dopaminergique avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avérerait effectivement posséder une affinité pour ce polypeptide,
- la détection de la formation éventuelle d'un complexe du type ligand-polypeptide.

L'invention concerne également un procédé pour l'étude de l'affinité d'un polypeptide de l'invention pour un ou plusieurs ligands déterminés, lequel procédé comprend :

- la transformation d'un hôte cellulaire compétent avec un vecteur, notamment un plasmide ou un phage, dans lequel avait auparavant été insérée une séquence de nucléotides codant pour le récepteur D-3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,

- la culture de l'hôte cellulaire transformé dans des conditions permettant l'expression dudit insérat, et le transport du récepteur D-3 exprimé vers la membrane de façon telle que les séquences transmembranaires du récepteur D-3 soient exposées à la surface de l'hôte cellulaire transformé,
- la mise en contact de cet hôte cellulaire avec ces ligands déterminés,
- la détection d'une liaison spécifique entre ledit hôte cellulaire transformé et lesdits ligands déterminés.

Le procédé décrit ci-dessus permet également l'identification des "fragments contenant les sites" dont question précédemment et qui ne comportent qu'une partie de la séquence de 446 acides aminés de la Figure 1 ou de la Figure 2.

Cette identification consiste à utiliser des insérats de taille plus réduite que l'acide nucléique codant pour la susdite séquence, à mettre en oeuvre les étapes relatives à l'expression, au transport du produit d'expression et à l'exposition rappelées ci-dessus. Lorsqu'on obtient l'expression, le transport du produit d'expression et son exposition sur la membrane, ainsi que la réaction avec les ligands telle que précédemment définie, on peut déterminer ainsi les fragments contenant les sites essentiels. Par conséquent, l'absence de réaction avec les ligands, telle que précédemment définie, en utilisant des fragments de taille plus réduite que la séquence complète, tendrait à montrer qu'on a éliminé certains sites essentiels.

L'invention concerne également une trousse pour la détection de l'affinité éventuelle d'un ligand pour un polypeptide de l'invention, laquelle trousse comprend:

- une culture d'hôtes cellulaires transformés par un vecteur modifié tel que défini précédemment ou une culture d'hôtes cellulaires définis précédemment, ou une préparation de membranes desdits hôtes,
- des moyens physiques ou chimiques pour induire l'expression de la séquence nucléotidiques contenue dans le vecteur modifié lorsque le promoteur placé en amont de cette séquence est un promoteur inductible par lesdits moyens physiques ou chimiques, et obtenir une protéine,
- un ou plusieurs ligands témoins ayant des affinités déterminées pour le susdit polypeptide,
- des moyens physiques ou chimiques pour la caractérisation de l'activité biologique de la protéine exprimée.

L'invention concerne également un procédé de criblage de médicaments destinés au traitement d'affections neurologiques (telles que la maladie de Parkinson), psychiatriques (telles que les états psychotiques), cardio-vasculaires (telles que l'hypertension artérielle) ou neuroendocriniennes (telles que les dysfonctionnements de l'axe hypothalamo-hypophysaire).

L'invention concerne également des médicaments contenant, comme substance active, une substance active sur l'un au moins des polypeptides selon l'invention, ayant une activité de récepteur dopaminergique ou contenant comme substance active des substances actives sur des cellules transfectées par les gènes ou les fragments de gènes codant l'un au moins des polypeptides à activité de récepteur dopaminergique selon l'invention, en association avec un véhicule pharmaceutiquement acceptable.

L'invention concerne également des médicaments contenant, comme substance active, une substance active sur les récepteurs dopaminergiques D-1 ou D-2, mais inactive sur les polypeptides à activité de récepteur dopaminergique selon l'invention.

Comme membrane biologique, on peut également travailler sur des membranes de rein.

Le ligand marqué et sélectif vis-à-vis d'un polypeptide de l'invention peut également être marqué par une méthode enzymatique.

En ce qui concerne la détection du complexe du type ligand sélectif marqué-polypeptide, elle a lieu selon des procédés classiques.

L'invention concerne également un procédé de marquage sélectif d'un polypeptide de l'invention à l'aide d'un ligand marqué selon une technique radioisotopique, sur des membranes biologiques contenant d'une part le récepteur D-1 et/ou D-2 et d'autre part le susdit polypeptide, comprenant les étapes suivantes :

- s'il y a lieu, la détermination d'un ligand sélectif vis-à-vis du récepteur D-1 et/ou D-2 selon le procédé décrit à propos de l'étude de l'affinité d'un polypeptide de l'invention,
- la mise en contact, selon la technique décrite dans Martres, M.P., Bouthenet, M.L., Salès, N., Sokoloff, P. et Schwartz, J.C. Science 228, 752-755 (1985), des susdites membranes biologiques avec le susdit ligand sélectif vis-à-vis du récepteur D-1 et/ou D-2 et avec un ligand marqué selon une technique radioisotopique, notamment l'iodosulpride-$^{125}$I,

ce qui conduit au marquage sélectif du susdit polypeptide, le récepteur D-1 et/ou D-2 étant occupé par le susdit ligand sélectif,

- la détection de la formation du complexe du type ligand marqué-polypeptide.

La détection du complexe du type ligand marqué-polypeptide peut se faire par des techniques classiques.

Le ligand marqué peut également être marqué selon une méthode enzymatique.

Ce procédé repose sur l'utilisation d'un ligand marqué (par une technique radioisotopique ou enzymatique), lequel ligand est non sélectif vis-à-vis du récepteur D-3, ce qui implique que si les récepteurs D-1 et/ou D-2 sont également contenus dans les susdites membranes biologiques, ce ligand marqué est susceptible de se lier à la fois aux récepteurs D-1, D-2 et D-3.

Afin d'utiliser le ligand marqué dans des conditions telles que le marquage du récepteur D-3 soit sélectif, on a recours à un ligand sélectif vis-à-vis du récepteur D-2 et/ou D-1. Ceci a pour objet d'occuper le récepteur D-2 et/ou D-1 et de le (les) rendre indisponible(s) vis-à-vis du susdit ligand marqué. Il en résulte donc que le susdit ligand marqué ne peut plus qu'occuper le récepteur D-3, ce qui entraîne la formation d'un complexe du type ligand marqué-récepteur D-3.

Le récepteur D-3 présente une distribution anatomique et des caractéristiques pharmacologiques qui le distinguent des récepteurs D-1 et D-2. Il est exprimé préférentiellement dans les régions limbiques du cerveau impliquées dans les processus cognitifs et émotionnels alors que les récepteurs D-1 et D-2 sont présents dans la plupart des régions dopaminoceptives, notamment le système extrapyramidal impliqué dans la motricité involontaire.

La localisation anatomique très particulière du récepteur D-3 dans le système limbique laisse supposer que des agonistes sélectifs de ce récepteur puissent avoir des effets thérapeutiques sur certains troubles cognitifs et émotionnels associés à la maladie de Parkinson.

Les neuroleptiques utilisés en clinique pour traiter les schizophrénies ont tous une affinité pour le récepteur D-3 de l'ordre de 1 à 10 nM qui suggère que le blocage de ce récepteur participe probablement aux effets thérapeutiques des neuroleptiques. De plus, le rapport des affinités respectives des neuroleptiques pour les récepteurs D-2 et D-3 est exprimé par le rapport $K_iD\text{-}2/K_iD\text{-}3$ comme indiqué dans le tableau I ci-après.

Tableau 1 :

| Pharmacologie des récepteurs D-2 et D-3 exprimés dans des cellules CHO | | | |
|---|---|---|---|
| Agents | Valeur $K_i$ (nM) | | Rapport $\frac{K_{iD2}}{K_{iD3}}$ |
| | récepteur D-2 | récepteur D-3 | |
| Agonistes | | | |
| Bromocriptine | 5.3 ± 0.6 | 7.4 ± 1.3 | 0.7 |
| Apomorphine | 24 ± 2 | 20 ± 3 | 1.2 |
| SKF 38393 | 9,560 ± 408 | 5,000 ± 500 | 1.9 |
| Dopamine | 474 ± 33 | 25 ± 3 | 19 |
| Dopamine + Gpp(NH)p | 1,705 ± 270 | 27 ± 3 | 63 |
| TL 99 | 18 ± 1 | 0.91 ± 0.08 | 20 |
| Pergolide | 21 ± 3 | 0.62 ± 0.04 | 33 |
| Quinpirole | 576 ± 47 | 5.1 ± 0.3 | 113 |
| Antagonistes | | | |
| Domperidone | 0.30 ± 0.03 | 9.5 ± 0.5 | 0.032 |
| Haloperidol | 0.45 ± 0.03 | 9.8 ± 0.3 | 0.046 |
| Spiperone | 0.069 ± 0.002 | 0.61 ± 0.05 | 0.11 |
| Thioproperazine | 0.21 ± 0.01 | 1.7 ± 0.1 | 0.12 |
| Prochlorperazine | 4.7 ± 0.4 | 35 ± 3 | 0.13 |
| (+)Sulpiride | 85 ± 7 | 422 ± 19 | 0.20 |
| Clozapine | 56 ± 2 | 180 ± 17 | 0.31 |
| (-)Sulpiride | 9.2 ± 0.3 | 25 ± 1 | 0.36 |
| Thioridazine | 3.3 ± 0.2 | 7.8 ± 0.8 | 0.42 |
| Amisulpride | 1.7 ± 0.1 | 3.8 ± 0.3 | 0.45 |
| Chlorpromazine | 2.8 ± 0.2 | 6.1 ± 0.3 | 0.46 |
| Raclopride | 1.8 ± 0.1 | 3.5 ± 0.3 | 0.51 |
| Iodosulpride* | 0.61 ± 0.05* | 1.2 ± 0.2* | 0.52 |

Tableau 1 : (suite)

| Pharmacologie des récepteurs D-2 et D-3 exprimés dans des cellules CHO | | | |
|---|---|---|---|
| Agents | Valeur $K_i$ (nM) | | Rapport $\frac{K_{iD2}}{K_{iD3}}$ |
| | récepteur D-2 | récepteur D-3 | |
| Pimozide | 2.4 ± 0.3 | 3.7 ± 0.5 | 0.65 |
| (+)AJ 76 | 270 ± 14 | 91 ± 5 | 3.0 |
| (+)UH 232 | 40 ± 1 | 9.2 ± 0.1 | 4.4 |

Les valeurs de $K_i$ sont dérivées d'expériences décrites dans la légende des Figures 5a et 5b. Les courbes ont été analysées par la méthode de régression non linéaire à l'aide d'un calculateur (Martres M.P. et al. "Selection of dopamine antagonists discriminating various behavioural responses and radioligand binding sites" Naunyn-Schmiedeberg's Arch. Pharmacol., 1984, 325:102-115). Les valeurs de $*K_D$ dérivent de cinétique de saturation à l'équilibre. Les moyennes ± l'écart moyen au standard de valeurs correspondant à 2-3 expériences, avec des résultats similaires. Les termes agonistes et antagonistes se réfèrent aux actions des médicaments vis-à-vis des récepteurs $D_2$. Les composés sont rangés selon leur rapport $K_i$D-2/$K_i$-D3.

SKF 38393 est décrit dans Pendleton R.G. et al. "Studies on renal dopamine receptors with SK&F 38393, a new agonist" Eur. J. Pharmacol., 1978, 51:19-28, AJ 76 et UH 232 sont décrits dans Svensson K. et al.

"(+)- AJ 76 and (+)-UH 232 : central stimulants acting as preferential dopamine autoreceptor antagonists" Naunyn-Schmiedeberg's Arch. Pharmacol., 1986, 334:234-245.

TL99 est décrit dans Goodale D.B. et al. Neurochemical and behavioral evidence for a selective presynaptic dopamine receptor agonist. Science, 1980, 210, 1141-1143.

Ce rapport est corrélé avec certaines propriétés cliniques des neuroleptiques : ceux qui présentent un faible rapport, d'environ 0,05 à environ 0,13 (halopéridol, thiopropérazine, prochlorpérazine) sont des neuroleptiques pour lesquels l'incidence des effets secondaires (parkinsonisme, dystonie et dyskinésies tardives) est la plus forte alors que ceux dont le rapport est le plus élevé, d'environ 0,3 à environ 0,6 (clozapine, amisulpride, thioridazine, sulpiride) sont des neuroleptiques "atypiques" qui provoquent moins d'effets secondaires et/ou ont des propriétés désinhibitrices dans certaines formes déficitaires de schizophrénie.

Le criblage de neuroleptiques potentiels par comparaison de leurs affinités respectives pour les récepteurs D-2 et D-3 fournit donc un test prédictif de leurs propriétés "atypiques" ce qui n'existait pas jusqu'alors.

D'autres caractéristiques et avantages de l'invention apparaissent dans la suite de la description et des exemples, notamment en rapport avec les dessins et tableaux dans lesquels :

- La Figure 1 représente les séquences d'acides aminés du récepteur dopaminergique D-2 (deuxième ligne) de rat, alignée avec celle d'un variant du récepteur dopaminergique D-3 de rat (première ligne). Dans les séquences du récepteur dopaminergique D-2, les résidus d'amino acides identiques et en position analogue à ceux du variant du récepteur dopaminergique D-3 sont marqués par des doubles traits (=).

Pour mettre en évidence les homologies, on a effectué des délétions dans les deux séquences, et ces délétions sont représentées par les espaces pointillés (-).

Dans la région N-terminale extra-cellulaire du variant du récepteur D-3, les séquences consensus pour les sites de glycosylation liés à l'asparagine (NXS/T) sont en positions 12 et 19.

- La Figure 2 représente les séquences d'acides aminés du récepteur dopaminergique D-2 (deuxième ligne) de rat, alignée avec celle du récepteur dopaminergique D-3 de rat (première ligne) selon Bunzow J.R. et al. ("Cloning and expression of a rat D-2 dopamine receptor cDNA", Nature, 1988, 336:783-787).

Les régions transmembranaires sont encadrées et les positions des introns pour le récepteur D-2A du rat (20, 22, 30, 33) et D-3, déduites de l'analyse de λRGE 12A et λRGS 3, sont indiquées par des flèches. Chaque signe "égal" indique une conservation en amino acides et les "tirets" correspondent aux substitutions conservatives où les groupes sont (H,K,R), (I,L,V,M), (A,G,S,T), (Y,F,W), (D,E,P,N,B,Z), (P) et (C).

- La Figure 3 représente la séquence d'acides nucléiques du susdit variant (cf. Figure 1) du récepteur dopaminergique D-3 chez le rat, et la séquence d'acides aminés correspondante.
- La Figure 4 représente la séquence d'acides nucléiques du récepteur dopaminergique D-3 chez le rat, et la séquence d'acides aminés correspondante.

- La Figure 5 représente des analyses de Northern Blot d'ARN de tissus cérébraux de rat. Dans la Figure 5, les références 1 à 10 correspondent respectivement à :

  1 : hypothalamus,
  2 : medulla-pont,
  3 : bulbe olfactif,
  4 : tubercules olfactifs,
  5 : striatum,
  6 : substance noire,
  7 : cortex,
  8 : hippocampe,
  9 : cervelet,
  10 : hypophyse.

- La Figure 6 représente schématiquement l'organisation des clones obtenus après le criblage de l'ADN génomique et l'amplification par technique PCR.

La séquence D-3 est schématiquement représentée par sept cases correspondant à MbI-MbVII, les lignes ouvertes représentant des séquences codantes et les lignes uniques les séquences non traduites.

λ RGE 12A et λ RGS 3 sont des clones d'ADN génomique, tandis que RT-PCR A et B représentent des clones d'ADNc obtenus à partir d'amplification selon la technique PCR d'ADNc obtenu à partir de poly(A)* ARNm et de réverse transcriptase ci-après désignée par RT-PCR. Les lignes sombres représentent les régions codantes (exons de clones d'ADN génomique) et les lignes fines représentent les introns dans les clones d'ADN génomique. Les lignes en pointillés indiquent la continuité de la séquence. Les amorces 1, 2 et 3 sont utilisées pour la technique RT-PCR. Les sites de restriction indiqués sont tels que: A = AvaI; Ba = BamHI; Bg = BglII; Bs = Bst XI; E = EcoRI; N = NheI et S = SalI. A noter que EcoRI, BglI et SalI sont des adaptateurs de l'extrémité 5' des amorces de RT-PCR.

- Les Figures 7a et 7b représentent les propriétés comparées des récepteurs D-2 et D-3 exprimés dans les cellules transfectées CHO. Plus précisément, la Figure 7a représente l'inhibition de la liaison à l'iodosulpride-$^{125}$I par les agonistes de la dopamine et la Figure 7b représente l'inhibition de la liaison à l'iodosulpride-$^{125}$I par les antagonistes de la dopamine.
- Les Figures 8a et 8b représentent la caractérisation des transcripts du gène du récepteur D-3 dans différents tissus.
- La Figure 9 représente la localisation autoradiographique des sites de liaison à l'iodosulpride-$^{125}$I et de l'ARN messager des récepteurs D-2 ou D-3 dans le cerveau de rat.
- La Figure 10 représente les effets des lésions induites par la 6-hydroxydopamine sur les transcripts des gènes des récepteurs D-2 et D-3 dans la substance noire.
- La Figure 11 représente la liaison spécifique aux récepteurs $D_2$ (courbe avec des cercles) et $D_3$ (courbe avec des points noirs) en présence de 7 OHDPAT-$^3$P en concentrations croissantes. Le 7 OHDPAT-$^3$P se lie à des membranes de cellules exprimant le récepteur $D_3$ avec une haute affinité ($K_D$ - 2 nM) mais très peu à celles de cellules exprimant le récepteur $D_2$, ce qui démontre la spécificité du ligand pour le récepteur $D_3$.

EXEMPLES

**1) Clonage du récepteur dopaminergique D-3 :**

Des oligonucléotides dérivés du récepteur dopaminergique D-2B (nucléotides 146-233 Bunzow, J.R., Van Tol, H. H.M., Grandy, D.K., Albert, P., Salon, J., Christie, Mc. D., Machida, C.A., Neve, K.A., and Civelli, O. Nature 336. 783-787 (1988)) sont marqués radioactivement et utilisés pour cribler une banque d'ADNc de cerveau de rat (Giros, B., Sokoloff, P., Martres, M.P., Riou, J.F., Emorine, L.J. et Schwartz, J.C. Nature 342. 923-926 (1989)). On montre que l'un des clones s'hybride faiblement avec un fragment de restriction BamHI-BglII du clone du récepteur D-2A mais pas avec une sonde AccI-AccI (nucléotides 278-571) codant pour les régions transmembranaires MbII à MbV représentées sur la Figure 2. Un fragment AatII-MscI de ce clone, marqué au phosphore 32 selon la technique de "nick-translation", est utilisé pour cribler une banque génomique de rat, partiellement coupée par EcoRI construite dans le vecteur Charon 4A. Des filtres de nitrocellulose sur lesquels ont été transférés des phages sont soumis à une hybridation 16 h à 42°C dans un milieu contenant 60% de formamide, 10% de sulfate de dextran, 4 x SSC, Tris-HCl 8 mM, pH 7,4, 1 x Denhardt's, 20μg/ml d'ARNt de levure, 20μg/ml d'ADN de sperme de saumon, 0,1% de SDS, et la sonde à 7,5 $10^5$dpm/ml. On lave les filtres deux fois pendant 20mn à 42°C dans 2 x SSC, 0,1% de SDS et deux fois pendant 30mn dans 0,2 x SSC, 0,1% de SDS à 42°C et 55°C respectivement. Trois clones identiques positifs sont obtenus (y compris λ RGE 12A) parmi 800.000 clones et un fragment NheI-NheI de 3,5kb est souscloné dans le site XbaI de M13 et séquencé (Sanger, F., Nicklen, S. et Coulson, A.R. Proc. Natl. Acad. Sci. USA, 74, 5463-5467 (1977)). On synthétise deux amorces dérivées de cette séquence (synthétiseur d'ADN PCR-Mate 391A, Applied Biosystems) à -32 (5'-ACATTTTGGAGTCGCGTTCCTCTG, amorce 4) et à -7 (CGAATTCATGGCACCTCTGAGCCAGATAAGCAC, amorce 1) et une amorce dégénérée de la séquence de MbVII du récepteur D2 (amorce 2) (5'-CGAATTCGAC(GA)GCACTGTT(GC)AC (GA)TA(GC)CC(GC)AGCCA) à 375nM.

On synthétise un ADNc monobrin en utilisant de la réverse transcriptase AMV (20 U, Boehringer), et du poly(A)* mARN (2μg) de cerveau de rat. Cette matrice est amplifiée (Kawazaki, E.S. et Wang, P.M. In : PCR Technology. (ed Erlich, H.A.) 89-97 (Stockholm Press. 1989)) en utilisant l'amorce 4 et l'amorce 2 (75nM de chacun) pendant 30 cycles (92°C, 52°C et 72°C pendant lmn chacun) avec 2,5U d'ADN polymérase Taq (Perkin Elmer Cetus).

Après résolution des produits qui ont été obtenus par la technique PCR par électrophorèse sur gel d'agarose, transfert et hybridation avec la sonde du récepteur D-2 AccI-AccI, une bande réactive est excisée à 1,3kb, et on extrait l'ADN. Cet ADN est amplifié à nouveau par technique PCR avec les amorces 1 et 2. Une bande est détectée après coloration au bromure d'éthidium, coupée, l'ADN est extrait et digéré par EcoRI et souscloné dans M13 (clone RT-PCR A) pour séquençage, et dans le plasmide pGEM-4Z (Promega). Un fragment de restriction marqué radioactivement Bst XI-EcoRI est utilisé pour cribler une bande génomique de rat, partiellement coupée par Sau 3A (Clontech) comme décrit ci-dessus. Un clone positif RGS3 est obtenu, analysé et un fragment NheI-NheI de 3,5kb hybridant avec la sonde Bst XI-EcoRI est souscloné dans M13 et séquencé. Une amorce est synthétisée 45 nucléotides en aval du premier codon stop TGA dans le cadre (amorce 3: CCACGTCGACAGATCTCGAAGTGGGTAAAGGGAGTG). Les amorces 1 et 3 sont ensuite utilisées dans la technique RT-PCR comme décrit ci-dessus, utilisant le poly(A)* ARNm de tubercule olfactif comme source d'ARN.

Une bande de taille prévue (1,4kb) est extraite après électrophorèse sur gel d'agarose et digérée avec EcoRI-SalI pour sousclonage directionnel dans M13 mp 18, M13 mp 19 et pGEM 4Z. Quatre clones individuels dans M13 sont entièrement séquencés et s'avèrent être identiques.

**2) Expression du récepteur D-3 dans des cellules CHO transfectées :**

On a effectué l'inhibition de la liaison iodosulpride-$^{125}$I par les agonistes de la dopamine (Figure 7a) et les antagonistes (Figure 7b).

METHODE

Les vecteurs d'expression dérivent du plasmide pSV $\beta_2$-MDH (Emorine, L., Marullo, S., Delavier-Klutchko, C., Kaveri, S.V., Durieu-Trautmann, O. et Strosberg, A.D. Proc. Natl. Acad. Sci. USA 84, 6995-6999 (1987)). Le récepteur adrénergique $\beta_2$ est excisé de pSV $\beta_2$-MDH par digestions avec HindIII et EcoRI et remplacé par un fragment de restriction SmaI-EcoRI du clone du récepteur D-2A en utilisant un adaptateur de nucléotide franc HindIII conduisant au plasmide pSV D-2. Le vecteur pour exprimer le récepteur D-3 est obtenu en remplaçant le fragment de restriction HindIII-BglII de pSV D-2 par un fragment de restriction EcoRI-BglII du clone RT-PCR B, en utilisant un adaptateur d'oligonucléotide HindIII-EcoRI conduisant au plasmide pSV D-3. Ces constructions sont cultivées et purifiées sur gradient de chlorure de cesium (Sambrook J. et al., Molecular cloning - a laboratory manual. C. Nolan, ed., Cold Spring Harbor Laboratory Press, 1989) et transfectées (Graham, F.L. et Van der Eb, A.J. Virology 52, 456-467 (1973)) à des cellules d'ovaire de hamster chinois (CHO-K1) déficientes en dihydrofolate réductase. Les transfectants stables sont sélectionnés dans un milieu de culture (Dubelcco's Modified Eagle medium) sans hypoxanthine et sans thymidine.

L'expression du récepteur D-3 est mise en évidence par la liaison de l'iodosulpride-$^{125}$I à des membranes de cellules transfectées selon la technique de Martres et col. 1985. Les expériences de liaison sont effectuées (Martres, M.P., Bouthenet, M.L., Salés, N., Sokoloff, P. et Schwartz, J.C. Science 228, 752-755 (1985)) dans un tampon Tris-HCl 50mM (volume total 400μl) contenant NaCl 120mM, KCl 5mM, $CaCl_2$ 2 mM, $MgCl_2$ 2mM, 0,1% d'acide ascorbique, 8-hydroxy quinoléine 10μM, 0,1% d'albumine de sérum bovin et iodosulpride-$^{125}$I 0,1 à 0,2nM dans les expériences avec des récepteurs D-2 et D-3, respectivement.

RESULTATS

Les récepteurs D-2 et D-3 ont été exprimés dans des cellules COS-7 ou des cellules CHO, qui normalement n'ont pas de sites de liaison vis-à-vis du d'iodosulpride-$^{125}$I, qui est un ligand présentant une haute affinité vis-à-vis des récepteurs D-2 mais pas du récepteur D-1 dans le cerveau. Les récepteurs D-2 et D-3 sont l'un et l'autre marqués au-dessus d'un seuil de liaison non spécifique.

Dans des clones transfectés en permanence de cellules CHO, les valeurs de $K_D$ sont de 0,6nM et 1,2nM respectivement pour D-2 et D-3, et les valeurs Bmax (Bmax représentant la concentration maximale respectivement en récepteur D-2 et D-3) sont d'environ 1pmol/mg de protéine de membrane dans les deux cas.

On peut distinguer clairement les deux récepteurs à l'aide de plusieurs agonistes et antagonistes de la dopamine. La dopamine elle-même, en l'absence de guanylnucléotide ajouté est d'environ 20 fois plus affine vis-à-vis du récepteur D-3 que vis-à-vis du récepteur D-2 (cf. Tableau 1 et Figures 7a et 7b).

En présence de Gpp(NH)p, un guanylnucléotide, la courbe de déplacement de la dopamine vis-à-vis du récepteur D-2 est transférée à droite (Figure 7a) indiquant la présence d'une protéine G appropriée. Au contraire, la courbe de déplacement de la dopamine vis-à-vis du récepteur D-3 n'est pas modifiée de façon significative en présence de guanylnucléotide (Figure 7b). Ceci peut correspondre à l'absence d'une protéine G appropriée dans la cellule CHO ainsi que dans les cellules COS-7. Cela peut aussi correspondre à une modulation faible de la liaison de la dopamine par les guanylnucléotides vis-à-vis du récepteur D-3.

Parmi les agonistes de la dopamine, l'apomorphine et la bromocriptine montrent des affinités similaires vis-à-vis des deux récepteurs, tandis que TL99 et le pergolide, considérés comme des agents sélectifs autorécepteurs sélectifs présumés (Clark, D. and White, F.J. Synapse 1, 347-388 (1987) ; Wolf, M.E. and Roth, R.H. In : Dopamine receptors. vol. 8 (eds Creese, I. and Fraser, C.M.) 45-96 (Alan R. Liss Inc. New-York, 1987)) et le quinpirole sont beaucoup plus affins vis-à-vis du récepteur D-3 que vis-à-vis du récepteur D-2.

La plupart des antagonistes de la dopamine appartenant à des classes chimiques variées et cliniquement utilisés comme neuroleptiques montrent des affinités nanomolaires vis-à-vis des deux récepteurs. Par exemple le halopéridol, le spiperone, la thioproperazine, et la prochlorperazine sont approximativement 10 à 20 fois plus affins vis-à-vis du récepteur D-2 que vis-à-vis du récepteur D-3 tandis que le sulpiride (-), la clozapine, la thioridazine, l'amisulpride ou le raclopride sont seulement 2 à 3 fois plus affins vis-à-vis du récepteur D-2 que vis-à-vis du récepteur D-3.

Les seuls antagonistes qui présentent des affinités limitées mais significativement plus élevées vis-à-vis du récepteur D-3 que vis-à-vis de D-2, sont UH232 et AJ76 (défini ci-après), considérés comme des agents présumés sélectifs des autorécepteurs.

**3) Caractérisation des transcripts du gène du récepteur D-3 dans plusieurs tissus (cf. Figure 8a et Figure 8b):**

Sur la Figure 8a, on a représenté l'analyse par transfert des ARN messagers du récepteur D-3 à partir de cerveau de rat. Pour ce faire, des ARNs poly(A)$^+$ sont préparés (Chirgwin, J.J., Przbyla, A.E., MacDonald, R.J. et Rutter, W.J. Biochemistry 18, 5294-5299 (1979) ; Aviv, H. et Leder, P., Proc. Natl. Acad. Sci. USA 69, 1408-1412 (1972)). Des échantillons (8µg) sont dénaturés, soumis à électrophorèse sur agarose, transférés sur des membranes de nitrocellulose et hybridés (Giros, B., Sokoloff, P., Martres, M.P., Riou, J.F., Emorine, L.J. et Schwartz, J.C. Nature 342. 923-926 (1989)) en utilisant un fragment sélectif de 422pb de restriction BamHI-AvaI marqué au $^{32}$P par la technique de "nick-translation", lequel fragment est situé dans la troisième boucle présumée intracytoplasmique, à 5 x $10^6$dpm/ml. Les membranes sont exposées pendant 6 jours à -80°C avec des écrans amplificateurs. Les tailles des marqueurs d'ARN (en kb) sont représentées sur la gauche de la Figure 8a.

On a représenté sur la Figure 8b, les autoradiogrammes d'électrophorèse sur gel d'agarose de produits d'ADNc marqués au phosphore 32, générés à partir de poly(A)* ARNm par technique PCR après amplification de 25 cycles (haut) et amplification de 32 cycles (bas). L'ADNc monobrin est synthétisé en utilisant 1µg d'ARN, 20U de transcriptase réverse AMV et l'amorce 3 (Figure 2). L'ADNc double brin est synthétisé et amplifié en utilisant 2,5U de polymérase Taq, l'amorce 3 et un oligonucléotide dérivé des amino acides 300-306 (Figure 2) : 5'-AAGCGCTACTACAGCATCTGC (amorce 5) pour 25 ou 32 cycles (92°C, 56°C et 72°C, lmn chacun). Une dose traceuse de ($^{32}$P)dCTP est incorporée pendant l'amplification et l'ADN est soumis à une électrophorèse dans de l'agarose à 1,5%.

**4) Localisation autoradiographique des sites de liaison iodosulpride $^{125}$I et des ARNm des récepteurs D-2 ou D-3 dans le cerveau de rat :**

Cette localisation fait l'objet de la Figure 9.

Les sections sagittales (A,B,C) ou coronales (D,E,F) (latéralité 0.4mm, niveau interaural 10.7mm (Paxinos, G. et Watson, C. In : The rat brain stereotaxic coordinates (Academic Press, London. 1982)) sont incubées avec soit de l'iodosulpride $^{125}$I (A,D), une sonde à la fois pour les récepteurs D-2 ou D3, soit avec des sondes d'ARN marquées au phosphore 32 sélectives vis-à-vis de l'ARNm du récepteur D-2 (B,E) ou vis-à-vis de l'ARNm du récepteur D-3 (C,F).

Les abréviations utilisées sur la Figure 9 sont les suivantes : Acb : nucleus accumbens; Cl : claustrum; Cg : cortex cingule; CM: noyau thalamique médian central; CPu : caude putamen; Fr : cortex frontal; G : noyau thalamique gélatineux; Hip : formation hippocampale; Icj : ilots de Calleja; ICJM : ilot de Calleja majeur; MM : partie médiane du noyau mammillaire médian; MP : partie postérieure du noyau mammillaire médian; MPA : aire préoptique médiane; OB: bulbe olfactif; PF : noyau thalamique parafasciculaire; Tu : tubercule olfactif; TT : ténia tecta; VTA : aire tegmentale ventrale; VI : couche VI du cortex cérébral.

La liaison avec l'iodosulpride $^{125}I$ est effectuée sur des sections de cryostat non fixées incubées avec 0,3nM d'iodosulpride $^{125}I$, rincées et apposées pendant 5 jours sur une pellicule désignée sous le nom $^{3}H$-hyperfilm d'Amersham (Bouthenet, M.L., Martres M.P., Salès, N. et Schwartz J.C. Neuroscience 20, 117-155 (1987)). Pour les études d'hybridation in situ, on synthétise des sondes d'ARN monobrin marquées au $^{32}P$ en utilisant une trousse commercialisée sous le nom Riboprobe (Promega) avec $^{32}P$-UTP et de l'ARN polymérase T7. Les matrices (10ng) sont des plasmides recombinants de pGEM-4Z digérés avec EcoRI contenant un fragment SacI-AflII du clone du récepteur D-2 ou un fragment BamHI-AvaI du clone B RT-PCR dans l'orientation appropriée. Des sections de cryostat (10µm) sont montées sur des plaques, fixées dans du formaldéhyde à 4%, traitées avec la protéinase K et sont soumises à l'hybridation une nuit à 55°C (Meador-Woodruff, J.H., Mansour, A., Bunzow, J.R., Van Tol, H.H.M., Watson, S.J. et Civelli, O. Proc. Natl. Acad. Sci. USA 86, 7625-7628 (1989)) avec des sondes marquées au $^{32}P$ à $2.10^{6}$ dpm dans 50µl. Les plaques sont rincées, déshydratées (Meador-Woodruff, J.H., Mansour, A., Bunzow, J.R., Van Tol, H.H.M., Watson, S. J. et Civelli, O. Proc. Natl. Acad. Sci. USA 86, 7625-7628 (1989)) et apposées sur des pellicules désignées sous le nom "βmax hyperfilm" (Amersham) pendant 20 jours à -80°C.

**5) Effets des lésions induites par la 6-hydroxydopamine sur les transcripts des gènes des récepteurs D-2 et D-3 dans la substance noire :**

Les rats reçoivent une injection unilatérale de 6-hydroxydopamine (8µg/4µl de solution saline) dans le faisceau médian du télencéphale aux coordonnées A 4,7mm, L 1,5mm et H 1,0mm (Paxinos, G. et Watson, C. In: The rat brain stereotaxic coordinates (Academic Press, London. 1982)). Après 10 jours, les rats présentant des rotations controlatérales suite à une administration de 0,25mg/kg d'apomorphine sont choisis. On les sacrifie 4 à 7 semaines après la lésion et on prépare l'ARN total de la substance noire individuelle ou de l'aire tegmentale ventrale (Chomczynski, P. et Sacchi, N. Analytical Biochemistry 162, 156-159 (1987)). On effectue les expériences de PCR comme décrites à propos des Figures 8a et 8b, en utilisant des oligonucléotides dérivés des amino acides 43-51 : 5'-GATGGTGGGTATGGGTCAGAAGGA et amino acides 243-253 : 5'-GCTCATTGCCGATAGTGATGACCT pour la β-actine. Pour le récepteur D-2, on utilise des oligonucléotides dérivés des amino acides 195-203 : 5'-TCCGAATTCTCATTCTACGTGCCCTTCATC et des amino acides 355-363 : 5'-GCTTTCTGCGGCTCATCGTCTTAA. Les amorces 3 et 5 (Figure 8) sont utilisées pour le récepteur D-3.

Les produits PCR sont obtenus après 16 cycles (rangées 1 et 4), 23 cycles (rangées 2 et 5) et 27 cycles (rangées 3 et 6) pour la β-actine et après 23 cycles (rangées 1 et 4), 27 cycles (rangées 2 et 5) et 30 cycles (rangées 3 et 6) pour les récepteurs D-2 et D-3 avec la substance noire d'un rat (rangées 1-3 : côté lésion; rangées 4-6: côté témoin). L'expérience est répétée 4-7 fois avec des tissus individuels et des expériences parallèles sont conduites avec l'aire tegmentale ventrale. On procède aux autoradiogrammes de gel, correspondant à 23 et 30 cycles pour la β-actine et les récepteurs respectivement. On calcule le rapport des densités optiques correspondant aux récepteurs et à la β-actine. Les rapports obtenus pour le côté de la lésion sont ensuite comparés aux rapports obtenus pour le côté témoin dans chacun des animaux.

**6) Détermination d'un ligand spécifique vis-à-vis du récepteur D-3 :**

L'hydroxy-7 N,N'dipropylaminotétraline radiomarqué au tritium (7 OHDPAT-$^{3}H$) a été incubé avec des membranes de cellules CHO exprimant, soit le récepteur D-2 soit le récepteur D-3 et le 7 OHDPAT-$^{3}H$ lié séparé du libre par filtration sous vide.

La Figure 11 représente la liaison spécifique aux récepteurs D-2 et D-3 en présence de 7 OHDPAT-$^{3}H$ en concentrations croissantes. Le 7 OHDPAT-$^{3}H$ se lie à des membranes de cellules exprimant le récepteur D-3 avec une haute affinité ($K_D$ = 2 nM) mais très peu à celles de cellules exprimant le récepteur D-2, ce qui démontre la spécificité du ligand pour le récepteur D-3.

De tels ligands sélectifs des récepteurs D-3 pourraient être obtenus en utilisant les molécules suivantes : quinpirole, pergolide, TL 99, quinérolane (décrit dans Foreman M.M. et al., 1989, "Preclinical studies on quinerolane, a potent and highly selective D-2-dopaminergic agonist", J. Pharm. Exp. Ther., 250:227-235) et SND 919 (décrit dans Yamada K. et al., 1990, "Possible involvement of differing classes of dopamine D-2 receptors in yawning and stereotypy in rats", Psychopharmacology, 100:141-144).

## Revendications

**1.** Polypeptide ayant une activité de récepteur dopaminergique contenant :

- la séquence de 446 acides aminés de la Figure 1,

. ou un fragment de cette séquence, ce fragment étant tel que

* soit il contient néanmoins les sites contenus dans cette séquence et dont la présence est nécessaire pour que, lorsque ce fragment est exposé à la surface d'une cellule, il soit capable de lier la dopamine à ses agonistes ou antagonistes de manière spécifique et mesurable, par exemple au moyen d'un ligand radioactif selon la technique décrite dans Sokoloff P., Martres M.P. et Schwartz J.C. "Three classes of dopamine receptor (D-2, D-3, D-4) identified by binding studies with $^{3}$H-apomorphine and $^{3}$H-domperidone". Naunyn-Schmiedeberg's Arch. Pharmacol. 1980, 315: 89-102 et Martres M.P., Bouthenet M.L., Sales N., Sokoloff P. et Schwartz J.C. "Widespread distribution of brain dopamine receptors evidenced with $^{125}$I-iodosulpride, a highly selective ligand". Science, 1985, 228: 752-755,
* soit il est susceptible d'être reconnu par des anticorps qui reconnaissent également la susdite séquence de 446 acides aminés, mais ne reconnaissent ni le récepteur dopaminergique D-1, ni le récepteur dopaminergique D-2,
* soit il est susceptible de générer des anticorps reconnaissant la susdite séquence de 446 acides aminés mais ne reconnaissant ni le récepteur dopaminergique D-1, ni le récepteur dopaminergique D-2 ou polypeptides variants qui correspondent aux polypeptides sus-définis, comportant certaines mutations localisées, sans que les polypeptides ne perdent les propriétés de récepteur dopaminergique.

**2.** Polypeptide selon la revendication 1, contenant:

. la séquence de 446 acides aminés de la Figure 2,
. ou un fragment de cette séquence, ce fragment étant tel que

* soit il contient néanmoins les sites contenus dans cette séquence et dont la présence est nécessaire pour que, lorsque ce fragment est exposé à la surface d'une cellule, il soit capable de lier la dopamine à ses agonistes ou antagonistes de manière spécifique et mesurable, par exemple au moyen d'un ligand radioactif selon la technique décrite dans Martres, M.P., Bouthenet, M.L., Salès, N., Sokoloff, P. et Schwartz, J.C. Science 228, 752-755 (1985),
* soit il est susceptible d'être reconnu par des anticorps qui reconnaissent également la susdite séquence de 446 acides aminés, mais ne reconnaissent ni le récepteur dopaminergique D-1, ni le récepteur dopaminergique D-2,
* soit il est susceptible de générer des anticorps reconnaissant la susdite séquence de 446 acides aminés mais ne reconnaissant ni le récepteur dopaminergique D-1, ni le récepteur dopaminergique D-2.

**3.** Polypeptide selon la revendication 1, dont l'affinité des agonistes déterminée par compétition avec le ligand iodosulpride-$^{125}$I est décroissante dans l'ordre suivant :
quinpirole > apomorphine = dopamine.

**4.** Polypeptide selon la revendication 1 sont l'affinité des antagonistes déterminée par compétition avec le ligand iodosulpride-$^{125}$I est décroissante dans l'ordre suivant :
raclopride > UH 232 = halopéridol.

**5.** Polypeptide selon la revendication 1, caractérisé en ce qu'il est constitué par la séquence, représentée sur la Figure 1 ou sur la Figure 2, s'étendant de l'extrémité constituée par l'acide aminé en position 1 à celle constituée par l'extrémité en position 446.

**6.** Acide nucléique caractérisé en ce qu'il comprend ou est constitué par un enchaînement de nucléotides codant pour les polypeptides selon l'une quelconque des revendications 1 à 5.

**7.** Acide nucléique selon la revendication 5, caractérisé en ce qu'il comprend ou est constitué par l'enchaînement de nucléotides représenté sur la Figure 3 ou sur la Figure 4, s'étendant de l'extrémité constituée par le nucléotide 1 à celle constituée par le nucléotide 1863,
ou est constitué par l'enchaînement de nucléotides représenté sur la Figure 3 ou sur la Figure 4, s'étendant de l'extrémité constituée par le nucléotide 442 à celle constituée par le nucléotide 1779.

**8.** Vecteur recombinant, en particulier pour le clonage et/ou l'expression, notamment du type plasmide, cosmide, phage ou virus, caractérisé en ce qu'il contient un acide nucléique selon l'une des revendications 6 ou 7 en l'un de ses sites non essentiels pour sa réplication.

**9.** Vecteur recombinant selon la revendication 8, caractérisé en ce qu'il contient en l'un de ses sites non essentiels pour sa réplication des éléments nécessaires pour promouvoir l'expression d'une séquences d'acides aminés selon l'une des revendications 1 à 5, dans un hôte cellulaire et éventuellement un promoteur reconnu par les polymérases de l'hôte cellulaire, en particulier un promoteur inductible et éventuellement une séquence signal et une séquence d'ancrage.

**10.** Hôte cellulaire transformé par un vecteur recombinant selon l'une quelconque des revendications 8 ou 9 et comprenant les éléments de régulation permettant l'expression de la séquence nucléotidique codant pour le polypeptide selon l'une quelconque des revendications 1 à 5 dans cet hôte.

**11.** Hôte cellulaire transformé selon la revendication 10, caractérisé en ce qu'il est choisi parmi les bactéries, notamment E. coli.

**12.** Hôte cellulaire transformé selon la revendication 10, caractérisé en ce qu'il est choisi parmi les organismes eucaryotes tels que des cellules CHO ou COS-7.

**13.** Anticorps caractérisé(s) en ce qu'il(s) est (ou sont) dirigé(s) de façon spécifique contre un polypeptide selon l'une quelconque des revendications 1 à 5, et en ce qu'il(s) ne reconnait (ou reconnaissent) ni le récepteur dopaminergique D-1, ni le récepteur dopaminergique D-2 et en particulier celui ou ceux reconnaissant les séquences d'acides aminés suivantes:

- celle définie par la séquence d'acides aminés représentée sur la Figure 1 s'étendant de l'extrémité constituée par l'acide aminé en position 1 à celle constituée par l'acide aminé en position 38,
- celle définie par la séquence d'acides aminés représentée sur la Figure 1 s'étendant de l'extrémité constituée par l'acide aminé en position 135 à celle constituée par l'acide aminé en position 147,
- celle définie par la séquence d'acides aminés représentée sur la Figure 1 s'étendant de l'extrémité constituée par l'acide aminé en position 175 à celle constituée par l'acide aminé en position 187,
- celle définie par la séquence d'acides aminés représentée sur la Figure 1 s'étendant de l'extrémité constituée par l'acide aminé en position 210 à celle constituée par l'acide aminé en position 375,
- celle définie par la séquence d'acides aminés représentée sur la Figure 2 s'étendant de l'extrémité constituée par l'acide aminé en position 135 à celle constituée par l'acide aminé en position 147.

**14.** Sonde nucléotidique caractérisée en ce qu'elle s'hybride avec l'un des acides nucléiques selon les revendications 6 ou 7 ou leur séquence complémentaire dans les conditions d'hybridation telles qu'elle ne s'hybride pas aves les gènes ou ARN messager des récepteurs dopaminergiques D-1 ou D-2, et en particulier les sondes choisies parmi les sondes nucléotidiques suivantes :

. - celle définie par la séquence d'acides nucléiques représentée sur la Figure 3, s'étendant de l'extrémité constituée par le nucléotide en position 1, à celle constituée par le nucléotide en position 441,
. - celle définie par la séquence d'acides nucléiques représentée sur la Figure 3, s'étendant de l'extrémité constituée par le nucléotide en position 442 à celle constituée par le nucléotide en position 537,
. - celle définie par la séquence d'acides nucléiques représentée sur la Figure 3, s'étendant de l'extrémité constituée par le nucléotide en position 718, à celle constituée par le nucléotide en position 753,
. - celle définie par la séquence d'acides nucléiques représentée sur la Figure 3, s'étendant de l'extrémité constituée par le nucléotide en position 820, à celle constituée par le nucléotide en position 888,
. - celle définie par la séquence d'acides nucléiques représentée sur la Figure 3, s'étendant de l'extrémité constituée par le nucléotide en position 958, à celle constituée par le nucléotide en position 996,
. - celle définie par la séquence d'acides nucléiques représentée sur la Figure 3, s'étendant de l'extrémité constituée par le nucléotide en position 1068, à celle constituée par le nucléotide en position 1240,
. - celle définie par la séquence d'acides nucléiques représentée sur la Figure 3, s'étendant de l'extrémité constituée par le nucléotide en position 1068, à celle constituée par le nucléotide en position 1566,
. - celle définie par la séquence d'acides nucléiques représentée sur la Figure 4, s'étendant de l'extrémité constituée par le nucléotide en position 820, à celle constituée par le nucléotide en position 888,

ou leur séquence nucléotidique complémentaire.

**15.** Procédé de détection de la capacité d'une molécule à se comporter comme ligand vis-à-vis d'un polypeptide selon l'une quelconque des revendications 1 à 5, caractérisé par :

- la mise en contact de la molécule avec un hôte cellulaire préalablement transformé par un vecteur lui-même modifié par un insérat codant pour le susdit polypeptide, cet hôte portant à sa surface un ou plusieurs sites spécifiques de ce polypeptide, le cas échéant après induction de l'expression de cet insérat, cette mise en contact étant effectuée dans des conditions permettant la formation d'une liaison entre l'un au moins de ces sites spécifiques et ladite molécule dès lors qu'elle s'avérerait effectivement posséder une affinité pour ce polypeptide,
- la détection de la formation éventuelle d'un complexe du type ligand-polypeptide.

**16.** Procédé pour l'étude de l'affinité d'un polypeptide selon l'une quelconque des revendications 1 à 5, pour un ou plusieurs ligands déterminés, caractérisé par :

- la transformation d'un hôte cellulaire compétent avec un vecteur, notamment un plasmide ou un phage, dans lequel avait auparavant été insérée une séquence de nucléotides ou codant pour le récepteur D-3 (insérat), sous le contrôle d'éléments de régulation, notamment d'un promoteur reconnu par les polymérases de l'hôte cellulaire et permettant l'expression dans l'hôte cellulaire utilisé de ladite séquence de nucléotides,
- la culture de l'hôte cellulaire transformé dans des conditions permettant l'expression dudit insérat, et le transport du récepteur D-3 exprimé vers la membrane de façon telle que les séquences transmembranaires de récepteur D-3 soient exposées à la surface de l'hôte cellulaire transformé,
- la mise en contact de cet hôte cellulaire avec ces ligands déterminés,
- la détection d'une réaction affine entre ledit hôte cellulaire transformé et lesdits ligands déterminés.

**17.** Procédé de diagnostic in vitro de l'expression pathologique du récepteur dopaminergique D-3, dans lequel on utilise l'une ou plusieurs quelconques des sondes définies selon la revendication 14,
comprenant les étapes suivantes :

- l'amplification préalable possible des quantités de séquence nucléotidique selon l'une quelconque des revendications 6 ou 7 susceptible d'être contenue dans un échantillon biologique prélevé sur un patient, au moyen d'amorces d'ADN,
- la mise en contact de l'échantillon biologique indiqué ci-dessus avec une sonde nucléotidique selon la revendication 14,

dans des conditions permettant la production d'un complexe d'hybridation formé de ladite sonde et ladite séquence nucléotidique,

- la détection du complexe d'hybridation ci-dessus qui a pu se former.

**18.** Procédé de diagnostic in vitro d'anomalies génétiques, notamment polymorphismes du gène codant pour le récepteur dopaminergique D-3 chez un individu, susceptibles d'être corrélées à des affections psychiatriques, neurologiques, cardiovasculaires ou neuroendocriniennes, à partir d'un échantillon biologique, tel que le sang, prélevé chez un individu, selon un procédé comprenant les étapes suivantes :

- le traitement de l'acide nucléique issu de l'échantillon biologique sus-mentionné réalisé à l'aide d'une enzyme de restriction dans des conditions permettant l'obtention de fragments de restriction issus du clivage dudit acide nucléique au niveau de ces sites de restriction reconnus par ladite enzyme,
- la mise en contact d'une sonde nucléotidique selon la revendication 14, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

**19.** Procédé de diagnostic in vitro de détection de mutations ponctuelles de l'acide nucléique ou d'un fragment de l'acide nucléique codant pour le récepteur dopaminergique D-3 selon les revendications 6 ou 7, chez un individu, comprenant les étapes suivantes :

- la mise en contact d'une sonde nucléotidique selon la revendication 14, avec un prélèvement biologique, par exemple du sang, dans des conditions permettant le production éventuelle d'un complexe d'hybridation formé entre la sonde et l'acice nucléique ou le fragment d'acide nucléique sus-mentionné,

- la détection du susdit complexe d'hybridation,
- le séquençage de l'acide nucléique ou du fragment d'acide nucléique intervenant dans le susdit complexe d'hybridation,
- la comparaison de la séquence de l'acide nucléique ou du fragment d'acide nucléique intervenant dans le susdit complexe d'hybridation avec la séquence de l'acide nucléique (ne présentant pas de mutation) ou du fragment de l'acide nucléique (ne présentant pas de mutation) du récepteur dopaminergique D-3.

**20.** Procédé de diagnostic in vitro de l'expression pathologique du récepteur dopaminergique D-3, dans lequel on utilise l'un ou plusieurs anticorps selon la revendication 13 comprenant les étapes suivantes :

- la mise en contact d'un anticorps selon la revendication 13 avec le susdit prélèvement biologique dans des conditions permettant la production éventuelle d'un complexe immunologique formé entre le récepteur dopaminergique D-3 ou produit qui en a dérivé et l'anticorps de l'invention,
- la détection du susdit complexe immunologique formé.

**21.** Médicament contenant comme substance active une substance active sur l'un au moins des polypeptides selon l'une des revendications 1 à 5, ayant une activité de récepteur dopaminergique ou contenant comme substance active des substances actives sur des cellules transfectées par les gènes ou les fragments de gènes codant l'un au moins des polypeptides à activité de récepteur dopaminergique selon l'une des revendications 1 à 5 en association avec un véhicule pharmaceutiquement acceptable.

**22.** Procédé de marquage sélectif d'un polypeptide selon l'une quelconque des revendications 1 à 5, à l'aide d'un ligand marqué selon une technique radioisotopique, sur des membranes biologiques contenant d'une part le récepteur D-1 et/ou D-2 et d'autre part un polypeptide selon l'une des revendications 1 à 5, comprenant les étapes suivantes:

- s'il y a lieu, la détermination d'un ligand sélectif vis-à-vis du récepteur D-1 et/ou D-2 selon le procédé décrit dans la revendication 16,
- la mise en contact, selon la technique décrite dans Martres, M.P., Bouthenet, M.L., Salés, N., Sokoloff, P. et Schwartz, J.C. Science 228, 752-755 (1985), des susdites membranes biologiques avec le susdit ligand sélectif vis-à-vis du récepteur D-1 et/ou D-2 et avec un ligand marqué selon une technique radioisotopique, notamment l'iodosulpride-$^{125}$I,

ce qui conduit au marquage sélectif du susdit polypeptide, le récepteur D-1 et/ou D-2 étant occupé par le susdit ligand sélectif,

- la détection de la formation du complexe du type ligand marqué-polypeptide.

## Patentansprüche

**1.** Polypeptid mit dopaminerger Rezeptor-Aktivität, enthaltend:

- die Sequenz von 446 Aminosäuren der Figur 1
- oder ein Fragment dieser Sequenz, wobei dieses Fragment derart ist, daß
    - * es entweder gleichwohl die in dieser Sequenz enthaltenen Stellen enthält, deren Vorliegen dafür erforderlich ist, daß, wenn dieses Fragment auf der Oberfläche einer Zelle präsentiert wird, es Dopamin an seine Agonisten oder Antagonisten binden kann auf spezifische und meßbare Weise, beispielsweise mittels eines radioaktiven Liganden gemäß dem Verfahren, das in Sokoloff, P., Martres, M.P. und Schwartz, J.C., "Three classes of dopamine receptor (D-2, D-3, D-4) identified by binding studies with $^{3}$H-apomorphine and $^{3}$H-domperidone", Naunyn-Schmiedeberg's Arch. Pharmacol. 1980, 315: 89-102 und Martres, M.P., Bouthenet, M.L., Sales, N., Sokoloff, P. und Schwartz, J.C. "Widespread distribution of brain dopamine receptors evidenced with $^{125}$I-iodosulpride, a highly selective ligand", Science, 1985, 228: 752-755, beschrieben ist,
    - * oder es durch Antikörper erkannt werden kann, die gleichfalls die vorstehend genannte Sequenz von 446 Aminosäuren erkennen, aber weder den dopaminergen Rezeptor D-1 noch den dopaminergen Rezeptor D-2 erkennen,

* oder es Antikörper erzeugen kann, die die vorstehend genannte Sequenz von 446 Aminosäuren erkennen, aber weder den dopaminergen Rezeptor D-1 noch den dopaminergen Rezeptor D-2 erkennen,

oder Polypeptidvarianten, die den vorstehend definierten Polypeptiden entsprechen, die bestimmte lokalisierte Mutationen tragen, ohne daß die Polypeptide ihre dopaminergen Rezeptoreigenschaften verlieren.

**2.** Polypeptid nach Anspruch 1, enthaltend:

- die Sequenz von 446 Aminosäuren der Figur 2
- oder ein Fragment dieser Sequenz, wobei dieses Fragment derart ist, daß

  * es entweder gleichwohl die in dieser Sequenz enthaltenen Stellen enthält, deren Vorliegen dafür erforderlich ist, daß, wenn dieses Fragment auf der Oberfläche einer Zelle präsentiert wird, es Dopamin an seine Agonisten oder Antagonisten binden kann auf spezifische und meßbare Weise, beispielsweise mittels eines radioaktiven Liganden gemäß dem Verfahren, das in Martres, M.P., Bouthenet, M.L., Sales, N., Sokoloff, P. und Schwartz, J.C., Science 228, 752-755 (1985) beschrieben ist,
  * oder es durch Antikörper erkannt werden kann, die gleichfalls die vorstehend genannte Sequenz von 446 Aminosäuren erkennen, aber weder den dopaminergen Rezeptor D-1 noch den dopaminergen Rezeptor D-2 erkennen,
  * oder es Antikörper erzeugen kann, die die vorstehend genannte Sequenz von 446 Aminosäuren erkennen, aber weder den dopaminergen Rezeptor D-1 noch den dopaminergen Rezeptor D-2 erkennen.

**3.** Polypeptid nach Anspruch 1,
gegenüber dem die Affinität der Agonisten, bestimmt durch Kompetition mit dem Ligand $^{125}$I-Iodsulprid, in der folgenden Reihenfolge abnimmt:
Quinpirol > Apomorphin = Dopamin.

**4.** Polypeptid nach Anspruch 1,
gegenüber dem die Affinität der Antagonisten, bestimmt durch Kompetition mit dem Ligand $^{125}$I-Iodsulprid, in der folgenden Reihenfolge abnimmt:
Raclioprid > UH 232 = Halopéridol.

**5.** Polypeptid nach Anspruch 1,
dadurch gekennzeichnet, daß es durch die in Figur 1 oder in Figur 2 dargestellte Sequenz, die sich von dem durch die Aminosäure an Position 1 gebildeten Ende bis zu jenem, das durch das Ende an Position 446 gebildet wird, erstreckt, gebildet wird.

**6.** Nukleinsäure,
dadurch gekennzeichnet, daß sie umfaßt oder gebildet wird durch eine Aneinanderreihung von Nukleotiden, die die Polypeptide nach einem der Ansprüche 1 bis 5 kodieren.

**7.** Nukleinsäure nach Anspruch 5,
dadurch gekennzeichnet, daß sie umfaßt oder gebildet wird durch die Aneinanderreihung von Nukleotiden, die in Figur 3 oder in Figur 4 dargestellt ist und die sich von dem durch das Nukleotid 1 gebildeten Ende bis zu jenem, das durch das Nukleotid 1863 gebildet wird, erstreckt.
oder gebildet wird durch die Aneinanderreihung von Nukleotiden, die in Figur 3 oder Figur 4 dargestellt ist und die sich von dem durch das Nukleotid 442 gebildeten Ende bis zu jenem, das durch das Nukleotid 1779 gebildet wird, erstreckt.

**8.** Rekombinanter Vektor, insbesondere für die Klonierung und/oder Expression, insbesondere vom Typ Plasmid, Cosmid, Phage oder Virus,
dadurch gekennzeichnet, daß er eine Nukleinsäure nach einem der Ansprüche 6 oder 7 in einer seiner Stellen, die für seine Replikation nicht essenziell sind, enthält.

**9.** Rekombinanter Vektor nach Anspruch 8,
dadurch gekennzeichnet, daß er in einer seiner für seine Replikation nicht essenziellen Stellen Elemente, die erforderlich sind, um die Expression einer Aminosäuresequenz nach einem der Ansprüche 1 bis 5 in einem zellulären Wirt zu fördern, und gegebenenfalls einen Promotor, der von den Polymerasen des zellulären Wirts erkannt wird, insbesondere einen induzierbaren Promotor, und gegebenenfalls eine Signalsequenz und eine Ankersequenz enthält.

**10.** Zellulärer Wirt, der durch einen rekombinanten Vektor nach einem der Ansprüche 8 oder 9, der die Regulationselemente umfaßt, die die Expression der Nukleotidsequenz, die das Polypeptid nach einem der Ansprüche 1 bis 5 kodiert, in diesem Wirt ermöglicht, transformiert worden ist.

**11.** Transformierter zellulärer Wirt nach Anspruch 10,
dadurch gekennzeichnet, daß er aus den Bakterien, insbesondere E. coli, ausgewählt ist.

**12.** Transformierter zellulärer Wirt nach Anspruch 10,
dadurch gekennzeichnet, daß er aus den eukaryotischen Organismen, wie CHO- oder COS-7-Zellen, ausgewählt ist.

**13.** Antikörper,
dadurch gekennzeichnet, daß dieser oder diese spezifisch gegen ein Polypeptid nach einem der Ansprüche 1 bis 5 gerichtet ist bzw. sind und daß dieser oder diese weder den dopaminergen Rezeptor D-1 noch den dopaminergen Rezeptor D-2 erkennt bzw. erkennen und wobei insbesondere der oder die die folgenden Aminosäuresequenzen erkennt bzw. erkennen:

- jene, die durch die in Figur 1 dargestellte Aminosäuresequenz, die sich von dem durch die Aminosäure an Position 1 gcbildeten Ende zu jenem, das durch die Aminosäure an Position 38 gebildet wird, erstreckt, definiert wird,
- jene, die durch die in Figur 1 dargestellte Aminosäuresequenz, die sich von dem durch die Aminosäure an Position 135 gebildeten Ende zu jenem, das durch die Aminosäure an Position 147 gebildet wird, erstreckt, definiert wird,
- jene, die durch die in Figur 1 dargestellte Aminosäuresequenz, die sich von dem durch die Aminosäure an Position 175 gebildeten Ende zu jenem, das durch die Aminosäure an Position 187 gebildet wird, erstreckt, definiert wird,
- jene, die durch die in Figur 1 dargestellte Aminosäuresequenz, die sich von dem durch die Aminosäure an Position 210 gebildeten Ende zu jenem, das durch die Aminosäure an Position 375 gebildet wird, erstreckt, definiert wird,
- jene, die durch die in Figur 2 dargestellte Aminosäuresequenz, die sich von dem durch die Aminosäure an Position 135 gebildeten Ende zu jenem, das durch die Aminosäure an Position 147 gebildet wird, erstreckt, definiert wird.

**14.** Nukleotidsondc,
gekennzeichnet dadurch, daß sie mit einer der Nukleinsäuren nach den Ansprüchen 6 oder 7 oder deren komplementärer Sequenz unter derartigen Hybridisierungsbedingungen hybridisiert, daß sie nicht mit den Genen oder der mRNA der dopaminergen Rezeptoren D-1 oder D-2 hybridisiert, und insbesondere die Sonden, die ausgewählt sind aus den folgenden Nukleotidsonden:

- jene, die durch die in Figur 3 dargestellte Nukleinsäuresequenz, die sich von dem durch das Nukleotid an Position 1 gebildeten Ende bis zu jenem, das durch das Nukleotid an Position 441 gebildet wird, erstreckt, definiert wird,
- jene, die durch die in Figur 3 dargestellte Nukleinsäuresequenz, die sich von dem durch das Nukleotid an Position 442 gebildeten Ende bis zu jenem, das durch das Nukleotid an Position 537 gebildet wird, erstreckt, definiert wird,
- jene, die durch die in Figur 3 dargestellte Nukleinsäuresequenz, die sich von dem durch gas Nukleotid an Position 718 gebildeten Ende bis zu jenem, das durch das Nukleotid an Position 753 gebildet wird, erstreckt, definiert wird,
- jene, die durch die in Figur 3 dargestellte Nukleinsäuresequenz, die sich von dem durch das Nukleotid an Position 820 gebildeten Ende bis zu jenem, das durch das Nukleotid an Position 888 gebildet wird, erstreckt, definiert wird,
- jene, die durch die in Figur 3 dargestellte Nukleinsäuresequenz, die sich von dem durch das Nukleotid an Position 958 gebildeten Ende bis zu jenem, das durch das Nukleotid an Position 996 gebildet wird, erstreckt, definiert wird,
- jene, die durch die in Figur 3 dargestellte Nukleinsäuresequenz, die sich von dem durch das Nukleotid an Position 1068 gebildeten Ende bis zu jenem, das durch das Nukleotid an Position 1240 gebildet wird, erstreckt, definiert wird,
- jene, die durch die in Figur 3 dargestellte Nukleinsäuresequenz, die sich von dem durch das Nukleotid an Position 1068 gebildeten Ende bis zu jenem, das durch das Nukleotid an Position 1566 gebildet wird, erstreckt, definiert wird,
- jene, die durch die in Figur 4 dargestellte Nukleinsäuresequenz, die sich von dem durch das Nukleotid an Position 820 gebildeten Ende bis zu jenem, das durch das Nukleotid an Position 888 gebildet wird, erstreckt, definiert wird,

oder deren komplementärer Nukleotidsequenz.

**15.** Verfahren zum Nachweis der Fähigkeit eines Moleküls, als Ligand gegenüber einem Polypeptid nach einem der Ansprüche 1 bis 5 zu wirken,
gekennzeichnet durch:

- das Inkontaktbringen des Moleküls mit einem zellulären Wirt, der vorab transformiert worden ist durch einen Vektor, der selbst durch ein Insert, das das vorstehend genannte Polypeptid kodiert, modifiziert worden ist, wobei dieser Wirt auf seiner Oberfläche eine oder mehrere spezifische Stellen dieses Polypeptids, gegebenenfalls nach Induktion der Expression dieses Inserts, trägt, wobei dieses Inkontaktbringen unter Bedingungen ausgeführt wird, die die Bildung einer Bindung zwischen mindestens einer dieser spezifischen Stellen und dem Molekül ermöglichen, sofern es sich herausstellt, daß dieses effektiv eine Affinität für dieses Polypeptid aufweist.
- den Nachweis der gegebenenfalls erfolgten Bildung eines Komplexes vom Typ Ligand-Polypeptid.

**16.** Verfahren zur Untersuchung der Affinität eines Polypeptids nach einem der Ansprüche 1 bis 5 für einen oder mehrere bestimmte Liganden,
gekennzeichnet durch:

- die Transformation eines kompetenten zellulären Wirts mit einem Vektor, insbesondere einem Plasmid oder einem Phagen, in den zuvor eine Nukleotidsequenz oder eine, die den Rezeptor D-3 kodiert, (Insert) unter der Kontrolle von Regulationselementen, insbesondere einem Promotor, der von den Polymerasen des zellulären Wirts erkannt wird und die Expression der Nukleotidsequenz in dem verwendeten zellulären Wirt ermöglicht, insertiert worden ist,
- die Züchtung des transformierten zellulären Wirts unter Bedingungen, die die Expression des Inserts und den Transport des exprimierten Rezeptors D-3 in Richtung auf die Membran dergestalt ermöglichen, daß die Transmembransequenzen des Rezeptors D-3 an der Oberfläche des transformierten zellulären Wirts präsentiert werden,
- das Inkontaktbringen dieses zellulären Wirts mit diesen bestimmten Liganden,
- den Nachweis einer Affinitätsreaktion zwischen dem transformierten zellulären Wirt und den bestimmten Liganden.

**17.** Verfahren zur in vitro-Diagnose der pathologischen Expression des dopaminergen Rezeptors D-3, bei dem man eine oder mehrere der in Anspruch 14 definierten Sonden einsetzt und das die folgenden Schritte umfaßt:

- die vorab erfolgende mögliche Amplifikation der Mengen an Nukleotidsequenz nach einem der Ansprüche 6 oder 7, die in einer einem Patienten entnommenen biologischen Probe enthalten sein können, mittels DNA-Startsequenzen,
- das Inkontaktbringen der vorstehend angegebenen biologischen Probe mit einer Nukleotidsonde nach Anspruch 14 unter Bedingungen, die die Produktion eines aus der Sonde und der Nukleotidsequenz gebildeten Hybridisierungskomplexes ermöglichen,
- den Nachweis des vorstehend genannten Hybridisierungskomplexes, der sich gebildet haben könnte.

**18.** Verfahren zur in vitro-Diagnose genetischer Anomalien, insbesondere von Polymorphismen des den dopaminergen Rezeptor D-3 kodierenden Gens bei einem Patienten, die mit psychiatrischen, neurologischen, kardiovaskulären oder neuroendokrinen Erkrankungen korreliert werden können, ausgehend von einer biologischen Probe, wie Blut, das einem Patienten abgenommen worden ist, gemäß einem Verfahren, das die folgenden Schritte umfaßt:

- die Behandlung der aus der vorstehend genannten biologischen Probe stammenden Nukleinsäure, die mittels eines Restriktionsenzyms unter Bedingungen ausgeführt wird, die die Gewinnung von Restriktionsfragmenten ermöglichen, die aus der Spaltung der Nukleinsäure auf Ebene dieser für das Enzym bekannten Restriktionsstellen stammen,
- das Inkontaktbringen einer Nukleotidsonde nach Anspruch 14, die mit den vorstehend genannten Fragmenten unter Bedingungen hybridisieren kann, die die gegebenenfalls erfolgende Erzeugung von Hybridisierungskomplexen zwischen der Sonde und den vorstehend genannten Restriktionsfragmenten ermöglichen,
- den Nachweis der vorstehend genannten Hybridisierungskomplexe,
- die Messung der Größe der gegebenenfalls vorliegenden polymorphen Restriktionsfragmente, die an den vorstehend genannten Hybridisierungskomplexen beteiligt sind.

**19.** in vitro-Diagnoseverfahren zum Nachweis von Punktmutationen der Nukleinsäure oder eines Fragments der Nukleinsäure, die den dopaminergen Rezeptor D-3 kodiert, nach den Ansprüchen 6 oder 7 bei einem Patienten, das die folgenden Schritte umfaßt:

- das Inkontaktbringen einer Nukleotidsonde nach Anspruch 14 mit einem biologischen Abstrich, beispielsweise von Blut, unter Bedingungen, die die gegebenenfalls erfolgende Erzeugung eines Hybridisierungskomplexes, der zwischen der Sonde und der Nukleinsäure oder dem vorstehend genannten Nukleinsäurefragment gebildet wird, ermöglichen,
- den Nachweis des vorstehend genannten Hybridisierungskomplexes,
- die Sequenzierung der Nukleinsäure oder des Nukleinsäurefragments, die an dem vorstehend genannten Hybridisierungskomplex beteiligt sind,
- den Vergleich der Sequenz der Nukleinsäure oder des Nukleinsäurefragments, die an dem vorstehend genannten Hybridisierungskomplex beteiligt sind, mit der Sequenz der Nukleinsäure (die keine Mutation aufweist) oder des Fragments der Nukleinsäure (das keine Mutation aufweist) des dopaminergen Rezeptors D-3.

**20.** Verfahren zur in vitro-Diagnose der pathologischen Expression des dopaminergen Rezeptors D-3, bei dem man einen oder mehrere Antikörper nach Anspruch 13 verwendet und das die folgenden Schritte umfaßt:

- das Inkontaktbringen eines Antikörpers nach Anspruch 13 mit dem vorstehend genannten biologischen Abstrich unter Bedingungen, die die gegebenenfalls erfolgende Erzeugung eines immunologischen Komplexes ermöglichen, der zwischen dem dopaminergen Rezeptor D-3 oder einem Produkt, das davon abgeleitet worden ist, und dem Antikörper der Erfindung gebildet wird.
- den Nachweis des gebildeten, vorstehend genannten, immunologischen Komplexes.

**21.** Arzneimittel. das als wirksame Substanz eine auf mindestens eines der Polypeptide nach einem der Ansprüche 1 bis 5, die eine dopaminerge Rezeptoraktivität aufweisen, wirksame Substanz oder als wirksame Substanz auf durch die Gene oder die Genfragmente, die mindestens eines der Polypeptide mit dopaminerger Rezeptoraktivität nach einem der Ansprüche 1 bis 5 kodieren, transfizierte Zellen wirksame Substanzen in Verbindung mit einem pharmazeutisch annehmbaren Träger enthält.

**22.** Verfahren zur selektiven Markierung eines Polypeptids nach einem der Ansprüche 1 bis 5 mittels eines markierten Liganden nach einer Radioisotopentechnik auf biologischen Membranen, die einerseits den Rezeptor D-1 und/oder D-2 und andererseits ein Polypeptid nach einem der Ansprüche 1 bis 5 enthalten, wobei das Verfahren die folgenden Schritte umfaßt:

- gegebenenfalls die Bestimmung eines gegenüber dem Rezeptor D-1 und/oder D-2 selektiven Liganden gemäß dem in Anspruch 16 beschriebenen Verfahren,
- das Inkontaktbringen der vorstehend genannten biologischen Membranen mit dem vorstehend genannten, gegenüber dem Rezeptor D-1 und/oder D-2 selektiven Liganden gemäß dem in Martres, M.P., Bouthenet, M. L., Sales, N., Sokoloff, P. und Schwartz, J.C., Science 228, 752-755 (1985) beschriebenen Verfahren und mit einem markierten Liganden gemäß einer Radioisotopentechnik, insbesondere $^{125}$I-Iodsulprid, was zur selektiven Markierung des vorstehend genannten Polypeptids führt, wobei der Rezeptor D-1 und/oder D-2 durch den vorstehend genannten selektiven Liganden besetzt wird,
- den Nachweis der Bildung des Komplexes vom Typ markierter Ligand-Polypeptid.

## Claims

1. Polypeptide having a dopaminergic receptor activity, comprising:

   - the sequence of 446 amino acids of Figure 1,
   - or a fragment of this sequence, this fragment being such that

     * either it nevertheless contains the sites contained in this sequence and whose presence is necessary in order that, when this fragment is exposed to the surface of a cell, it is capable of binding dopamine to its agonists or antagonists in a specific and measurable manner, for example by means of a radioactive ligand according to the technique described in Sokoloff P., Martres M.P. and Schwartz J.C. "Three classes of dopamine receptor (D-2, D-3, D-4) identified by binding studies with $^3$H-apomorphine and $^3$H-domperidone". Naunyn-Schmiedeberg' s Arch. Pharmacol. 1980, 315: 89-102 and Martres M.P., Bouthenet M.L., Sales N., Sokoloff P. and Schwartz J.C. "Widespread distribution of brain dopamine receptors evidenced with $^{125}$I-iodosulpride, a highly selective ligand". Science, 1985, 228: 752-755,
     * or it is capable of being recognized by antibodies which likewise recognize the abovementioned sequence of 446 amino acids, but do not recognize either the dopaminergic receptor D-1, or the dopaminergic receptor D-2,
     * or it is capable of generating antibodies recognizing the abovementioned sequence of 446 amino acids but not recognizing either the dopaminergic receptor D-1, or the dopaminergic receptor D-2 or variant polypeptides which correspond to the polypeptides defined above, having certain localized mutations, without the polypeptides losing the dopaminergic receptor properties.

2. Polypeptide according to Claim 1, comprising:

   - the sequence of 446 amino acids of Figure 2,
   - or a fragment of this sequence, this fragment being such that

     * either it nevertheless contains the sites contained in this sequence and whose presence is necessary in order that, when this fragment is exposed to the surface of a cell, it is capable of binding dopamine to its agonists or antagonists in a specific and measurable manner, for example by means of a radioactive ligand according to the technique described in Martres, M.P., Bouthenet, M.L., Sales, N., Sokoloff, P. and Schwartz, J.C. Science 228, 752-755 (1985),
     * or it is capable of being recognized by antibodies which likewise recognize the abovementioned sequence of 446 amino acids, but do not recognize either the dopaminergic receptor D-1, or the dopaminergic receptor D-2,
     * or it is capable of generating antibodies recognizing the abovementioned sequence of 446 amino acids but not recognizing either the dopaminergic receptor D-1, or the dopaminergic receptor D-2.

3. Polypeptide according to Claim 1, of which the affinity of the agonists determined by competition with the iodosulpride-$^{125}$I ligand is decreasing in the following order:
   quinpirole > apomorphine = dopamine.

4. Polypeptide according to Claim 1, of which the affinity of the agonists determined by competition with the iodosulpride-$^{125}$I ligand is decreasing in the following order:
   raclopride > UH 232 = haloperidol.

5. Polypeptide according to Claim 1, characterized in that it is formed by the sequence represented in Figure 1 or in Figure 2, extending from the end formed by the amino acid in position 1 to that formed by the amino acid in position 446.

6. Nucleic acid characterized in that it comprises or is formed by a chain of nucleotides coding for the polypeptides according to any one of Claims 1 to 5.

7. Nucleic acid according to Claim 5, characterized in that it comprises or is formed by the chain of nucleotides represented in Figure 3 or in Figure 4, extending from the end formed by the nucleotide 1 to that formed by the nucleotide 1863,
   or is formed by the chain of nucleotides represented in Figure 3 or in Figure 4, extending from the end formed by the nucleotide 442 to that formed by the nucleotide 1779.

**8.** Recombinant vector, in particular for cloning and/or expression, especially of the plasmid, cosmid, phage or virus type, characterized in that it contains a nucleic acid according to one of Claims 6 or 7 in one of its sites which is not essential for its replication.

**9.** Recombinant vector according to Claim 8, characterized in that it contains in one of its sites which is not essential for its replication elements necessary for promoting the expression of a sequence of amino acids according to one of Claims 1 to 5 in a cellular host and optionally a promoter recognized by the polymerases of the cellular host, in particular an inducible promoter, and optionally a signal sequence and an anchorage sequence.

**10.** Cellular host transformed by a recombinant vector according to either of Claims 8 or 9 and comprising the regulation elements allowing the expression of the nucleotide sequence coding for the polypeptide according to any one of Claims 1 to 5 in this host.

**11.** Cellular host transformed according to Claim 10, characterized in that it is chosen from amongst the bacteria, especially E.coli.

**12.** Cellular host transformed according to Claim 10, characterized in that it is chosen from amongst the eucaryotic organisms such as CHO or COS-7 cells.

**13.** Antibody(ies) characterized in that it(they) is (or are) directed specifically against a polypeptide according to any one of Claims 1 to 5, and in that it(they) do not recognize either the dopaminergic receptor D-1, or the dopaminergic receptor D-2 and in particular that or those recognizing the following amino acid sequences:

- that defined by the sequence of amino acids represented in Figure 1 extending from the end formed by the amino acid in position 1 to that formed by the amino acid in position 38,
- that defined by the sequence of amino acids represented in Figure 1 extending from the end formed by the amino acid in position 135 to that formed by the amino acid in position 147,
- that defined by the sequence of amino acids represented in Figure 1 extending from the end formed by the amino acid in position 175 to that formed by the amino acid in position 187,
- that defined by the sequence of amino acids represented in Figure 1 extending from the end formed by the amino acid in position 210 to that formed by the amino acid in position 375,
- that defined by the sequence of amino acids represented in Figure 2 extending from the end formed by the amino acid in position 135 to that formed by the amino acid in position 147.

**14.** Nucleotide probe characterized in that it hybridizes with one of the nucleic acids according to Claim 6 or 7 or their complementary sequence under hybridization conditions such that it does not hybridize with the genes or messenger RNA of the dopaminergic receptor D-1 or D-2, and in particular the probes chosen from amongst the following nucleotide probes:

- that defined by the sequence of nucleic acids represented in Figure 3, extending from the end formed by the nucleotide in position 1 to that formed by the nucleotide in position 441,
- that defined by the sequence of nucleic acids represented in Figure 3, extending from the end formed by the nucleotide in position 442 to that formed by the nucleotide in position 537,
- that defined by the sequence of nucleic acids represented in Figure 3, extending from the end formed by the nucleotide in position 718 to that formed by the nucleotide in position 753,
- that defined by the sequence of nucleic acids represented in Figure 3, extending from the end formed by the nucleotide in position 820 to that formed by the nucleotide in position 888,
- that defined by the sequence of nucleic acids represented in Figure 3, extending from the end formed by the nucleotide in position 958 to that formed by the nucleotide in position 996,
- that defined by the sequence of nucleic acids represented in Figure 3, extending from the end formed by the nucleotide in position 1068 to that formed by the nucleotide in position 1240,
- that defined by the sequence of nucleic acids represented in Figure 3, extending from the end formed by the nucleotide in position 1068 to that formed by the nucleotide in position 1566,
- that defined by the sequence of nucleic acids represented in Figure 4, extending from the end formed by the nucleotide in position 820 to that formed by the nucleotide in position 888,

or their complementary nucleotide sequence.

**15.** Method of detection of the capacity of a molecule to behave as a ligand with respect to a polypeptide according to any one of Claims 1 to 5, characterized by:

- the contacting of the molecule with a cellular host previously transformed by a vector itself modified by an insert coding for the abovementioned polypeptide, this host carrying on its surface one or more specific sites of this polypeptide, if necessary after induction of the expression of this insert, this contacting being carried out under conditions allowing the formation of a bond between at least one of these specific sites and the said molecule as it could effectively turn out to have an affinity for this polypeptide,
- the detection of the possible formation of a complex of the ligand-polypeptide type.

**16.** Method for the study of the affinity of a polypeptide according to any one of Claims 1 to 5 for one or more determined ligands characterized by:

- the transformation of a competent cellular host with a vector, especially a plasmid or a phage, in which had previously been inserted a nucleotide sequence or coding for the receptor D-3 (insert), under the control of regulation elements, especially a promoter recognized by the polymerases of the cellular host and allowing expression in the cellular host used of the said sequence of nucleotides,
- the culture of the transformed cellular host under conditions allowing the expression of the said insert, and the transport of the receptor D-3 expressed towards the membrane in such a way that the transmembrane sequences of receptor D-3 are exposed to the surface of the transformed cellular host,
- the contacting of this cellular host with these determined ligands,
- the detection of an affinity reaction between the said transformed cellular host and the said determined ligands.

**17.** Method of in vitro diagnosis of the pathological expression of the dopaminergic receptor D-3, in which one or more of any of the probes defined according to Claim 14 are used,
comprising the following steps:

- the possible previous amplification of the quantities of nucleotide sequence according to either of Claims 6 and 7 capable of being contained in a biological sample taken from a patient, by means of DNA primers,
- the contacting of the biological sample indicated above with a nucleotide probe according to Claim 14, under conditions allowing the production of a hybridization complex formed from the said probe and the said nucleotide sequence,
- the detection of the above hybridization complex which has been able to form.

**18.** Method of in vitro diagnosis of genetic anomalies, especially polymorphisms of the gene coding for the dopaminergic receptor D-3 in an individual, capable of being correlated to psychiatric, neurologic, cardiovascular or neuroendocrine disorders, from a biological sample, such as blood, taken from an individual, according to a method comprising the following steps:

- the treatment of the nucleic acid from the abovementioned biological sample carried out with the aid of a restriction enzyme under conditions allowing the obtainment of restriction fragments from the cleavage of the said nucleic acid at the level of these restriction sites recognized by the said enzyme,
- the contacting of a nucleotide probe according to Claim 14, capable of hybridizing with the abovementioned fragments under conditions allowing the possible production of hybridization complexes between the said probe and the abovementioned restriction fragments,
- the detection of the abovementioned hybridization complexes,
- the size measurement of the possible polymorphic restriction fragments inserted in the abovementioned hybridization complexes.

**19.** Method of in vitro diagnosis for detection of point mutations of nucleic acid or of a fragment of nucleic acid coding for the dopaminergic receptor D-3 according to Claims 6 or 7, in an individual, comprising the following steps:

- the contacting of a nucleotide probe according to Claim 14 with a biological sample, for example blood, under conditions allowing the possible production of a hybridization complex formed between the probe and the nucleic acid or the abovementioned fragment of nucleic acid,
- the detection of the abovementioned hybridization complex,

- the sequencing of the nucleic acid or of the nucleic acid fragment taking place in the abovementioned hybridization complex,
- the comparison of the nucleic acid sequence or of the nucleic acid sequence fragment taking place in the abovementioned hybridization complex with the nucleic acid sequence (not having any mutation) or of the nucleic acid fragment (not having any mutation) of the dopaminergic receptor D-3.

**20.** Method of in vitro diagnosis of the pathological expression of the dopaminergic receptor D-3, in which one or more antibodies according to Claim 13 are used, comprising the following steps:

- the contacting of an antibody according to Claim 13 with the abovementioned biological sample under conditions allowing the possible production of an immunological complex formed between the dopaminergic receptor D-3 or product which has been derived from it and the antibody of the invention,
- the detection of the abovementioned immunological complex formed.

**21.** Medicament comprising as active substance a substance active on at least one of the polypeptides according to one of Claims 1 to 5, having a dopaminergic receptor activity or comprising as active substance substances active on cells transfected by the genes or the fragments of genes encoding at least one of the polypeptides having dopaminergic receptor activity according to one of Claims 1 to 5 in association with a pharmaceutically acceptable vehicle.

**22.** Method of selective labelling of a polypeptide according to any one of Claims 1 to 5, with the aid of a ligand labelled according to a radioisotopic technique, on biological membranes comprising on the one hand the receptor D-1 and/or D-2 and on the other hand a polypeptide according to one of Claims 1 to 5, comprising the following steps:

- if it is necessary, the determination of a selective ligand with respect to the receptor D-1 and/or D-2 according to the method described in Claim 16,
- the contacting, according to the technique described in Martres, M.P., Bouthenet, M.L., Sales, N., Sokoloff, P. and Schwartz, J.C. Science 228, 752-755 (1985), of the abovementioned biological membranes with the abovementioned selective ligand with respect to the receptor D-1 and/or D-2 and with a ligand labelled according to a radioisotopic technique, especially iodosulpride-$^{125}I$,
  which leads to the selective labelling of the abovementioned polypeptide, the receptor D-1 and/or D-2 being occupied by the abovementioned selective ligand,
- the detection of the formation of the complex of the type labelled ligand-polypeptide.

Figure 1

```
1         10         20           30        40        50        60
MAPLS-Q-ISTHLNSTCG-AENSTGVN-RA-RPH-AYYALSYCALILAIIFGNGLVCAAVLRERALQTTT
= ==        =-     -    = -=- ===  ===-    ==  =-=== === == ==-======
MDPLNLSWYDDDLERQ-NWSRPFNGSEGKADRPHYNYYAMLLTLLIFIIVFGNVLVCMAVSREKALQTTT
1       10         20        30        40        50        60

    70         80        90      100       110       120       130
NYLVVSLAVADLLVATLVMPWVVYLEVTGGVWNFSRICCDVFVTLDVMMCTASILNLCAISIDRYTAVVM
===-======================= =  = ==== ==-=========================== =
NYLIVSLAVADLLVATLVMPWVVYLEVVGE-WKFSRIHCDIFVTLDVMMCTASILNLCAISIDRYTAVAM
         80        90        100       110       120       130

   140       150       160       170       180       190       200
PVHYEHGTGQSSCRRVALMITAVWVLAFAVSCPLLFGFNTTGDPSICSISNPDFVIYSSVVSFYVPFGVT
=- =-   - =  ===-===- ====-=-======== = =-=   = =-== ==-===-======= ==
PMLYNTRYS-SK-RRVTVMIAIVWVLSFTISCPLLFGLNNT-DQNECIIANPAFVVYSSIVSFYVPFIVT
           150       160       170        180       190       200

   210       220       230         240       250       260        270
VLVYARIYIVLRQRQRKRILTRQNSQCISIRPGF--PQQSSCLRLHPIRQFSIRARFLSDATG-QMEHIE
-=== -====== = ===- =-  =    -=     =  - =   ==  -  - -  -   --  --
LLVYIKIYIVLRKR-RKRVNTKRSSR--AFRANLKTPLKGNC-T-HP-EDMKLCTVIMKSNGSFPVNR-R
  210        220       230         240          250       260
```

```
        280       290        300        310       320      330
DKQYPQKCQDPL-LSHLQPPSPGQ-TH-GGLKR-YYSIC-QDTALRHPSLEGGAGMSPVERTRNSLSPTM
 -   -==-=- =   ==-= -- -    -  =- =-=- -   ==   -=- - -
RMDAARRAQE-LEMEMLSSTSPPERTRYSPIPPSHHQLTLPDPS-HH-GLHSNPD-SPAKPEKNGHAKIV
            280       290       300       310        320       330

340        350      360        370      380       390      400
APKLS-L-EVRKLSNGRLSTSLRLGPLQPRGVPL-REKKATQMVVIVLGAFIVCWLPFFLTHVLNTHCQA
=---   =-  - ==-  ===- - -  = -   -=======- ====  ==-======-==-== ==-
NPRIAKFFEIQTMPNGKTRTSLK-T-MSRRKLSQQKEKKATQMLAIVLGVFIICWLPFFITHILNIHCD-
  340       350        360       370       380       390       400

  410       420       430      440
CHVSPELYRATTWLGYVNSALNPVIYTTFNVEFRKAFLKILSC    Récepteur D-3 (446 aa)
= - = == = ========-==-==========-======-=== =
CNIPPVLYSAFTWLGYVNSAVNPIIYTTFNIEFRKAFMKILHC    Récepteur D-2 (444 aa)
    410       420       430       440
```

Figure 1 suite

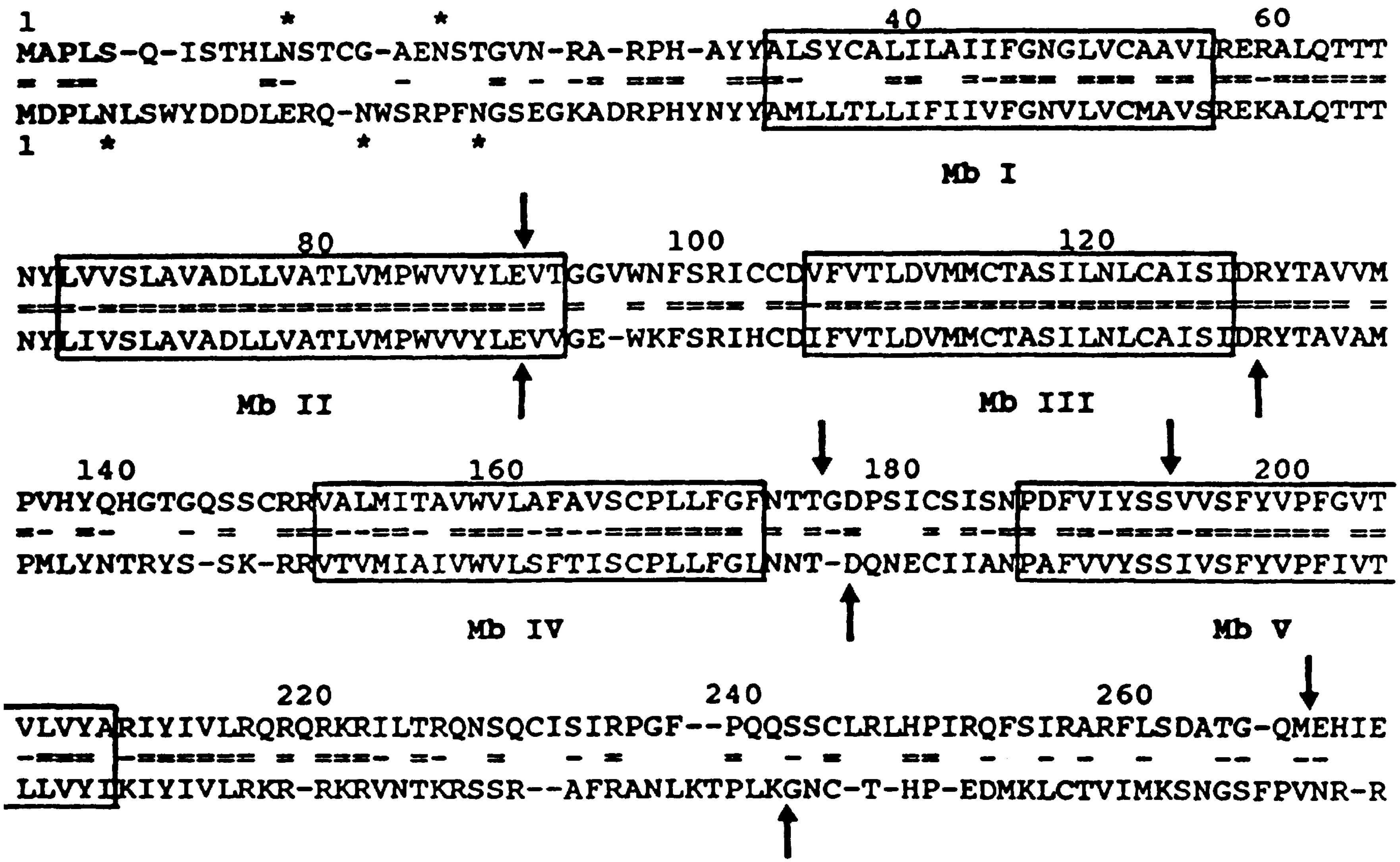
MAPLS-Q-ISTHLNSTCG-AENSTGVN-RA-RPH-AYYALSYCALILAIIFGNGLVCAAVIRERALQTTT
MDPLNLSWYDDDLERQ-NWSRPFNGSEGKADRPHYNYYAMLLTLLIFIIVFGNVLVCMAVSREKALQTTT
Mb I
NYLVVSLAVADLLVATLVMPWVVYLEVTGGVWNFSRICCDVFVTLDVMMCTASILNLCAISIDRYTAVVM
NYLIVSLAVADLLVATLVMPWVVYLEVVGE-WKFSRIHCDIFVTLDVMMCTASILNLCAISIDRYTAVAM
Mb II
Mb III
PVHYQHGTGQSSCRRVALMITAVWVLAFAVSCPLLFGFNTTGDPSICSISNPDFVIYSSVVSFYVPFGVT
PMLYNTRYS-SK-RRVTVMIAIVWVLSFTISCPLLFGLNNT-DQNECIIANPAFVVYSSIVSFYVPFIVT
Mb IV
Mb V
VLVYARIYIVLRQRQRKRILTRQNSQCISIRPGF--PQQSSCLRLHPIRQFSIRARFLSDATG-QMEHIE
LLVYIKIYIVLRKR-RKRVNTKRSSR--AFRANLKTPLKGNC-T-HP-EDMKLCTVIMKSNGSFPVNR-R

Figure 2

```
280                            300                            320
DKQYPQKCQDPL-LSHLQPPSPGQ-TH-GGLKR-YYSIC-QDTALRHPSLEGGAGMSPVERTRNSLSPTM
RMDAARRAQE-LEMEMLSSTSPPERTRYSPIPPSHHQLTLPDPS-HH-GLHSNPD-SPAKPEKNGHAKIV

340                     360                          380                 400
APKLS-L-EVRKLSNGRLSTSLRLGPLQPRGVPL-REKKATQMVVIVLGAFIVCWLPFFLTHVLNTHCQA
NPRIAKFFEIQTMPNGKTRTSLK-T-MSRRKLSQQKEKKATQMLAIVLGVFIICWLPFFITHILNIHCD-
                                                    Mb VI

          420                   446
CHVSPELYRATTWLGYVNSALNPVIYTTFNVEFRKAFLKILSC        Récepteur D-3
CNIPPVLYSAFTWLGYVNSAVNPIIYTTFNIEFRKAFMKILHC        Récepteur D-2
                                        444
            Mb VII
```

Figure 2 suite

```
gctagccttgccttcactgctaatatagccaggaagccttcttgttatctaa    52
tatagccaggaagccttcttgttatctaactgtgcttacccacaatcatacc   104
atcctcgaccactccccaactcccatttctgatttacttttctccaaaaagc   156
ataatatcgcagaacaggtcttatcttgattataaatcttctcccccccccc   208
caaccccatagaggtttcataagggaagaaatgtctgttcctttcctaactg   260
tatttctggttctatagcactgcctgctctatatagaaatgttccatcgata   312
tttgtagacatgaaacattttaaactgtatgtatgtaacatatcccagctct   364
gaagagcctgatttagcccacattgctgtctgtcttttcctaggaacatttt   416

ggagtcgcgttcctctgtgtgggcc ATG GCA CCT CTG AGC CAG      459
                          Met Ala Pro Leu Ser Gln        6

ATA AGC ACC CAC CTC AAC TCC ACC TGC GGG GCA GAA AAC    498
Ile Ser Thr His Leu Asn Ser Thr Cys Gly Ala Glu Asn     19

TCC ACT GGC GTC AAC CGG GCC CGT CCG CAC GCC TAC TAC    537
Ser Thr Gly Val Asn Arg Ala Arg Pro His Ala Tyr Tyr     32

GCC CTG TCC TAC TGT GCT CTC ATC CTA GCC ATC ATC TTT    576
Ala Leu Ser Tyr Cys Ala Leu Ile Leu Ala Ile Ile Phe     45

GGC AAC GGC CTG GTA TGT GCT GCT GTG CTG AGG GAG CGT    615
Gly Asn Gly Leu Val Cys Ala Ala Val Leu Arg Glu Arg     58

GCC CTG CAG ACC ACC ACC AAC TAC CTA GTG GTG AGC CTG    654
Ala Leu Gln Thr Thr Thr Asn Tyr Leu Val Val Ser Leu     71

GCT GTG GCC GAC CTG CTA GTG GCC ACG TTG GTG ATG CCG    693
Ala Val Ala Asp Leu Leu Val Ala Thr Leu Val Met Pro     84

TGG GTG GTG TAC TTG GAG GTG ACA GGT GGA GTC TGG AAT    732
Trp Val Val Tyr Leu Glu Val Thr Gly Gly Val Trp Asn     97

TTC AGC CGC ATT TGC TGT GAC GTT TTT GTC ACC CTG GAT    771
Phe Ser Arg Ile Cys Cys Asp Val Phe Val Thr Leu Asp    110

GTC ATG ATG TGT ACA GCC AGC ATC CTG AAC CTC TGT GCC    810
Val Met Met Cys Thr Ala Ser Ile Leu Asn Leu Cys Ala    123

ATC AGC ATA GAC AGG TAC ACA GCG GTG GTC ATG CCA GTT    849
Ile Ser Ile Asp Arg Tyr Thr Ala Val Val Met Pro Val    136

CAC TAT GAG CAC GGC ACC GGG CAG AGC TCC TGT AGA CGT    888
His Tyr Glu His Gly Thr Gly Gln Ser Ser Cys Arg Arg    149

GTG GCA CTC ATG ATC ACA GCT GTG TGG GTG CTG GCT TTT    927
Val Ala Leu Met Ile Thr Ala Val Trp Val Leu Ala Phe    162

GCT GTG TCC TGC CCT CTC CTC TTT GGT TTC AAC ACA ACA    966
Ala Val Ser Cys Pro Leu Leu Phe Gly Phe Asn Thr Thr    175

GGG GAT CCC AGC ATC TGC TCC ATC TCC AAC CCT GAT TTT   1005
Gly Asp Pro Ser Ile Cys Ser Ile Ser Asn Pro Asp Phe    188

GTC ATT TAC TCT TCA GTG GTG TCC TTC TAC GTT CCC TTC   1044
Val Ile Tyr Ser Ser Val Val Ser Phe Tyr Val Pro Phe    201
```

Figure 3

```
GGG GTG ACT GTC CTG GTC TAT GCC AGG ATC TAC ATA GTC  1083
Gly Val Thr Val Leu Val Tyr Ala Arg Ile Tyr Ile Val   214

CTG AGG CAA AGG CAA AGA AAA CGG ATC CTC ACT CGA CAG  1122
Leu Arg Gln Arg Gln Arg Lys Arg Ile Leu Thr Arg Gln   227

AAC AGC CAG TGC ATC AGT ATC AGA CCT GGC TTT CCT CAG  1161
Asn Ser Gln Cys Ile Ser Ile Arg Pro Gly Phe Pro Gln   240

CAG TCT TCC TGT CTG AGG CTG CAT CCC ATT CGG CAG TTT  1200
Gln Ser Ser Cys Leu Arg Leu His Pro Ile Arg Gln Phe   253

TCA ATA AGG GCC AGG TTT CTG TCA GAT GCC ACA GGA CAA  1239
Ser Ile Arg Ala Arg Phe Leu Ser Asp Ala Thr Gly Gln   266

ATG GAG CAC ATA GAA GAC AAA CAA TAT CCC CAG AAA TGC  1278
Met Glu His Ile Glu Asp Lys Gln Tyr Pro Gln Lys Cys   279

CAG GAC CCC CTT TTG TCA CAC CTG CAG CCC CCC TCA CCT  1317
Gln Asp Pro Leu Leu Ser His Leu Gln Pro Pro Ser Pro   292

GGT CAG ACA CAT GGG GGG CTG AAG CGC TAC TAC AGC ATC  1356
Gly Gln Thr His Gly Gly Leu Lys Arg Tyr Tyr Ser Ile   305

TGC CAA GAC ACT GCC TTG AGA CAC CCA AGC TTG GAA GGC  1395
Cys Gln Asp Thr Ala Leu Arg His Pro Ser Leu Glu Gly   318

GGG GCA GGG ATG AGC CCC GTG GAA AGG ACT CGG AAC TCC  1434
Gly Ala Gly Met Ser Pro Val Glu Arg Thr Arg Asn Ser   331

TTG AGC CCC ACC ATG GCA CCC AAG CTC AGC TTA GAG GTT  1473
Leu Ser Pro Thr Met Ala Pro Lys Leu Ser Leu Glu Val   344

CGA AAA CTC AGC AAC GGC AGG TTA TCC ACG TCC CTG AGG  1512
Arg Lys Leu Ser Asn Gly Arg Leu Ser Thr Ser Leu Arg   357

CTG GGG CCC CTG CAG CCT CGG GGA GTA CCA CTT CGA GAG  1551
Leu Gly Pro Leu Gln Pro Arg Gly Val Pro Leu Arg Glu   370

AAG AAG GCC ACC CAG ATG GTG GTC ATT GTG CTT GGA GCC  1590
Lys Lys Ala Thr Gln Met Val Val Ile Val Leu Gly Ala   383

TTC ATT GTC TGC TGG CTG CCC TTC TTC CTG ACT CAC GTT  1629
Phe Ile Val Cys Trp Leu Pro Phe Phe Leu Thr His Val   396

CTT AAT ACC CAC TGT CAA GCA TGC CAC GTG TCC CCA GAG  1668
Leu Asn Thr His Cys Gln Ala Cys His Val Ser Pro Glu   409

CTT TAC AGA GCC ACA ACG TGG CTA GGC TAT GTG AAC AGT  1707
Leu Tyr Arg Ala Thr Thr Trp Leu Gly Tyr Val Asn Ser   422

GCC CTG AAT CCT GTG ATC TAT ACC ACC TTC AAT GTG GAG  1746
Ala Leu Asn Pro Val Ile Tyr Thr Thr Phe Asn Val Glu   435

TTC CGC AAA GCC TTC CTC AAG ATC CTG TCC TGC  tgaagga 1786
Phe Arg Lys Ala Phe Leu Lys Ile Leu Ser Cys          446

ggagaagagaccgcactccctttacccacttcgagatgccaggcagtttgaa 1838
ccctgcccatcagggtctggttggg                            1863
```

Figure 3 (suite)

gctagccttgccttcactgctaatatagccaggaagccttcttgttatctaa 52
tatagccaggaagccttcttgttatctaactgtgcttacccacaatcatacc 104
atcctcgaccactccccaactcccatttctgatttactttctccaaaaagc 156
ataatatcgcagaacaggtcttatcttgattataaatcttctcccccccccc 208
caaccccatagaggtttcataagggaagaaatgtctgttcctttcctaactg 260
tatttctggttctatagcactgcctgctctatatagaaatgttccatcgata 312
tttgtagacatgaaacattttaaactgtatgtatgtaacatatcccagctct 364
gaagagcctgatttagcccacattgctgtctgtcttttcctaggaacatttt 416

ggagtcgcgttcctctgtgtgggcc ATG GCA CCT CTG AGC CAG 459
Met Ala Pro Leu Ser Gln 6

ATA AGC ACC CAC CTC AAC TCC ACC TGC GGG GCA GAA AAC 498
Ile Ser Thr His Leu Asn Ser Thr Cys Gly Ala Glu Asn 19

TCC ACT GGC GTC AAC CGG GCC CGT CCG CAC GCC TAC TAC 537
Ser Thr Gly Val Asn Arg Ala Arg Pro His Ala Tyr Tyr 32

GCC CTG TCC TAC TGT GCT CTC ATC CTA GCC ATC ATC TTT 576
Ala Leu Ser Tyr Cys Ala Leu Ile Leu Ala Ile Ile Phe 45

GGC AAC GGC CTG GTA TGT GCT GCT GTG CTG AGG GAG CGT 615
Gly Asn Gly Leu Val Cys Ala Ala Val Leu Arg Glu Arg 58

GCC CTG CAG ACC ACC ACC AAC TAC CTA GTG GTG AGC CTG 654
Ala Leu Gln Thr Thr Thr Asn Tyr Leu Val Val Ser Leu 71

GCT GTG GCC GAC CTG CTA GTG GCC ACG TTG GTG ATG CCG 693
Ala Val Ala Asp Leu Leu Val Ala Thr Leu Val Met Pro 84

TGG GTG GTG TAC TTG GAG GTG ACA GGT GGA GTC TGG AAT 732
Trp Val Val Tyr Leu Glu Val Thr Gly Gly Val Trp Asn 97

TTC AGC CGC ATT TGC TGT GAC GTT TTT GTC ACC CTG GAT 771
Phe Ser Arg Ile Cys Cys Asp Val Phe Val Thr Leu Asp 110

GTC ATG ATG TGT ACA GCC AGC ATC CTG AAC CTC TGT GCC 810
Val Met Met Cys Thr Ala Ser Ile Leu Asn Leu Cys Ala 123

ATC AGC ATA GAC AGG TAC ACA GCG GTG GTC ATG CCA GTT 849
Ile Ser Ile Asp Arg Tyr Thr Ala Val Val Met Pro Val 136

CAC TAT CAG CAC GGC ACC GGG CAG AGC TCC TGT AGA CGT 888
His Tyr Gln His Gly Thr Gly Gln Ser Ser Cys Arg Arg 149

GTG GCA CTC ATG ATC ACA GCT GTG TGG GTG CTG GCT TTT 927
Val Ala Leu Met Ile Thr Ala Val Trp Val Leu Ala Phe 162

GCT GTG TCC TGC CCT CTC CTC TTT GGT TTC AAC ACA ACA 966
Ala Val Ser Cys Pro Leu Leu Phe Gly Phe Asn Thr Thr 175

GGG GAT CCC AGC ATC TGC TCC ATC TCC AAC CCT GAT TTT 1005
Gly Asp Pro Ser Ile Cys Ser Ile Ser Asn Pro Asp Phe 188

GTC ATT TAC TCT TCA GTG GTG TCC TTC TAC GTT CCC TTC 1044
Val Ile Tyr Ser Ser Val Val Ser Phe Tyr Val Pro Phe 201

Figure 4

GGG GTG ACT GTC CTG GTC TAT GCC AGG ATC TAC ATA GTC 1083
Gly Val Thr Val Leu Val Tyr Ala Arg Ile Tyr Ile Val 214

CTG AGG CAA AGG CAA AGA AAA CGG ATC CTC ACT CGA CAG 1122
Leu Arg Gln Arg Gln Arg Lys Arg Ile Leu Thr Arg Gln 227

AAC AGC CAG TGC ATC AGT ATC AGA CCT GGC TTT CCT CAG 1161
Asn Ser Gln Cys Ile Ser Ile Arg Pro Gly Phe Pro Gln 240

CAG TCT TCC TGT CTG AGG CTG CAT CCC ATT CGG CAG TTT 1200
Gln Ser Ser Cys Leu Arg Leu His Pro Ile Arg Gln Phe 253

TCA ATA AGG GCC AGG TTT CTG TCA GAT GCC ACA GGA CAA 1239
Ser Ile Arg Ala Arg Phe Leu Ser Asp Ala Thr Gly Gln 266

ATG GAG CAC ATA GAA GAC AAA CAA TAT CCC CAG AAA TGC 1278
Met Glu His Ile Glu Asp Lys Gln Tyr Pro Gln Lys Cys 279

CAG GAC CCC CTT TTG TCA CAC CTG CAG CCC CCC TCA CCT 1317
Gln Asp Pro Leu Leu Ser His Leu Gln Pro Pro Ser Pro 292

GGT CAG ACA CAT GGG GGG CTG AAG CGC TAC TAC AGC ATC 1356
Gly Gln Thr His Gly Gly Leu Lys Arg Tyr Tyr Ser Ile 305

TGC CAA GAC ACT GCC TTG AGA CAC CCA AGC TTG GAA GGC 1395
Cys Gln Asp Thr Ala Leu Arg His Pro Ser Leu Glu Gly 318

GGG GCA GGG ATG AGC CCC GTG GAA AGG ACT CGG AAC TCC 1434
Gly Ala Gly Met Ser Pro Val Glu Arg Thr Arg Asn Ser 331

TTG AGC CCC ACC ATG GCA CCC AAG CTC AGC TTA GAG GTT 1473
Leu Ser Pro Thr Met Ala Pro Lys Leu Ser Leu Glu Val 344

CGA AAA CTC AGC AAC GGC AGG TTA TCC ACG TCC CTG AGG 1512
Arg Lys Leu Ser Asn Gly Arg Leu Ser Thr Ser Leu Arg 357

CTG GGG CCC CTG CAG CCT CGG GGA GTA CCA CTT CGA GAG 1551
Leu Gly Pro Leu Gln Pro Arg Gly Val Pro Leu Arg Glu 370

AAG AAG GCC ACC CAG ATG GTG GTC ATT GTG CTT GGA GCC 1590
Lys Lys Ala Thr Gln Met Val Val Ile Val Leu Gly Ala 383

TTC ATT GTC TGC TGG CTG CCC TTC TTC CTG ACT CAC GTT 1629
Phe Ile Val Cys Trp Leu Pro Phe Phe Leu Thr His Val 396

CTT AAT ACC CAC TGT CAA GCA TGC CAC GTG TCC CCA GAG 1668
Leu Asn Thr His Cys Gln Ala Cys His Val Ser Pro Glu 409

CTT TAC AGA GCC ACA ACG TGG CTA GGC TAT GTG AAC AGT 1707
Leu Tyr Arg Ala Thr Thr Trp Leu Gly Tyr Val Asn Ser 422

GCC CTG AAT CCT GTG ATC TAT ACC ACC TTC AAT GTG GAG 1746
Ala Leu Asn Pro Val Ile Tyr Thr Thr Phe Asn Val Glu 435

TTC CGC AAA GCC TTC CTC AAG ATC CTG TCC TGC tgaagga 1786
Phe Arg Lys Ala Phe Leu Lys Ile Leu Ser Cys 446

ggagaagagaccgcactccctttacccacttcgagatgccaggcagtttgaa 1838
ccctgcccatcagggtctggttggg 1863

Figure 4 (suite)

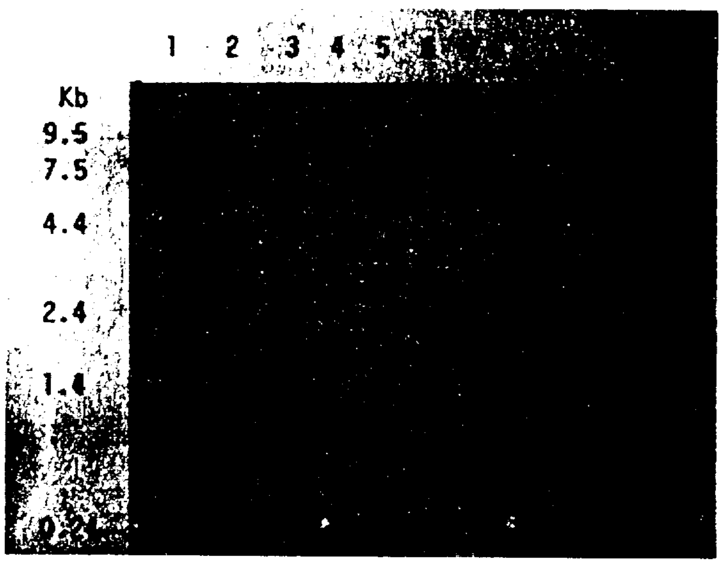


Figure 5

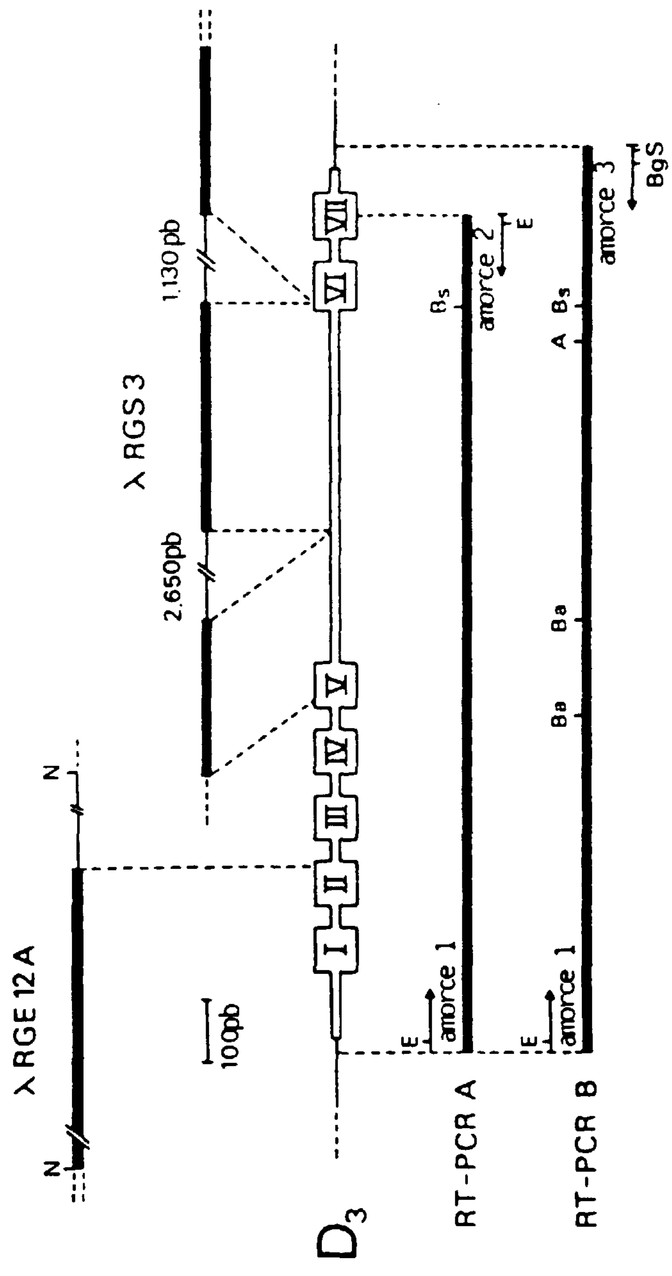
λ RGE 12 A
N
N
λ RGS 3
2.650pb
1.130pb
100pb
D3
I
II
III
IV
V
VI
VII
RT-PCR A
RT-PCR B
E
amorce 1
Bs
amorce 2
E
Ba
Ba
A
Bs
amorce 3
BgS

Figure 6

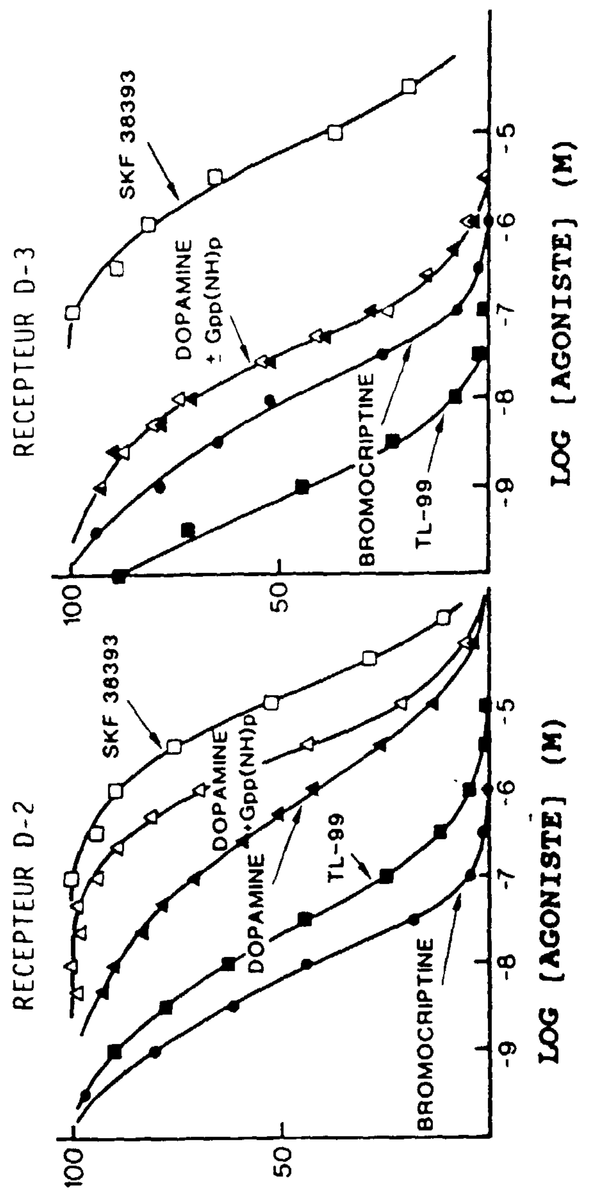


LIAISON SPECIFIQUE DE L'IODOSULPRIDE $I^{125}$ (%)

Figure 7a

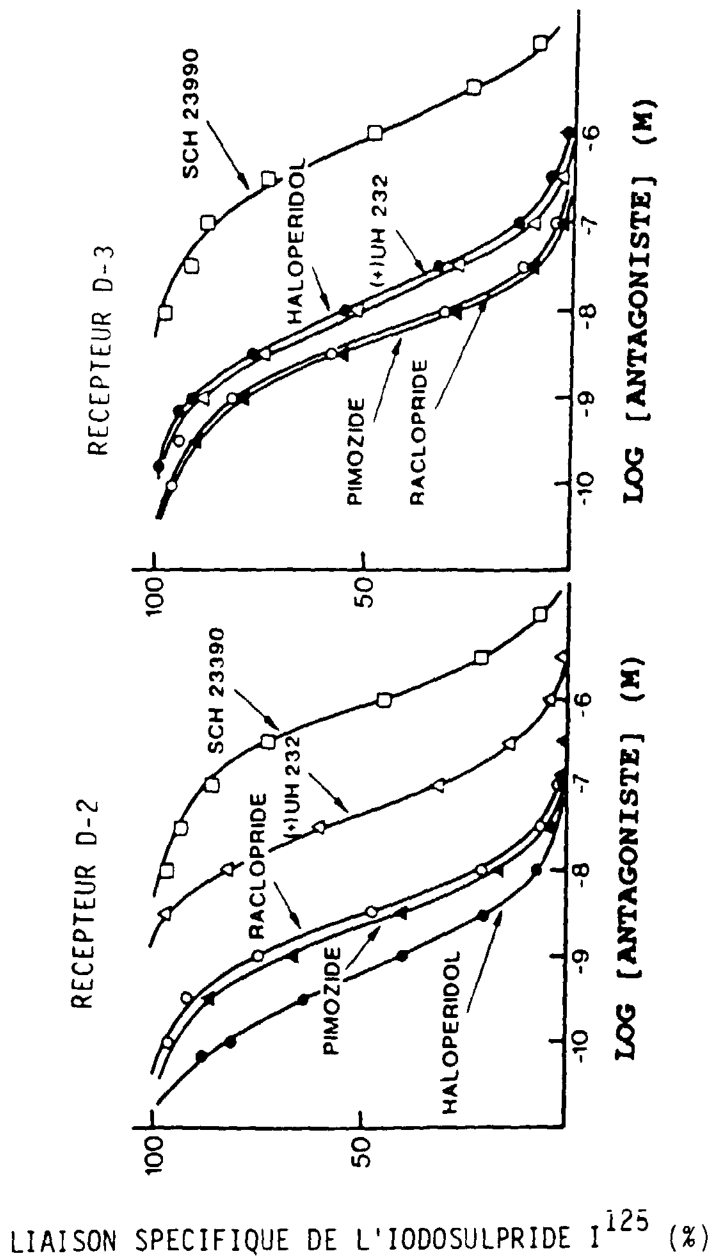
RECEPTEUR D-2
RECEPTEUR D-3
SCH 23390
(+)UH 232
RACLOPRIDE
PIMOZIDE
HALOPERIDOL
SCH 23990
HALOPERIDOL
(+)UH 232
PIMOZIDE
RACLOPRIDE
100
50
-10
-9
-8
-7
-6
LOG [ANTAGONISTE] (M)
LIAISON SPECIFIQUE DE L'IODOSULPRIDE I125 (%)

Figure 7b

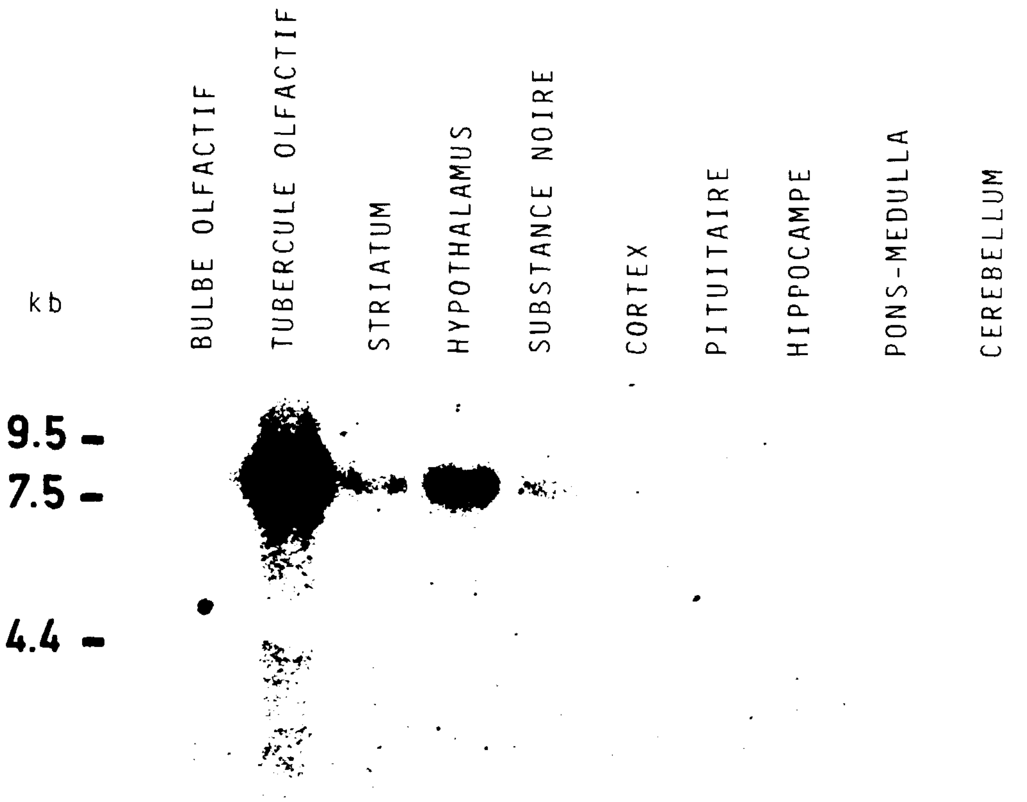
kb
BULBE OLFACTIF
TUBERCULE OLFACTIF
STRIATUM
HYPOTHALAMUS
SUBSTANCE NOIRE
CORTEX
PITUITAIRE
HIPPOCAMPE
PONS-MEDULLA
CEREBELLUM
9.5
7.5
4.4

Figure 8a

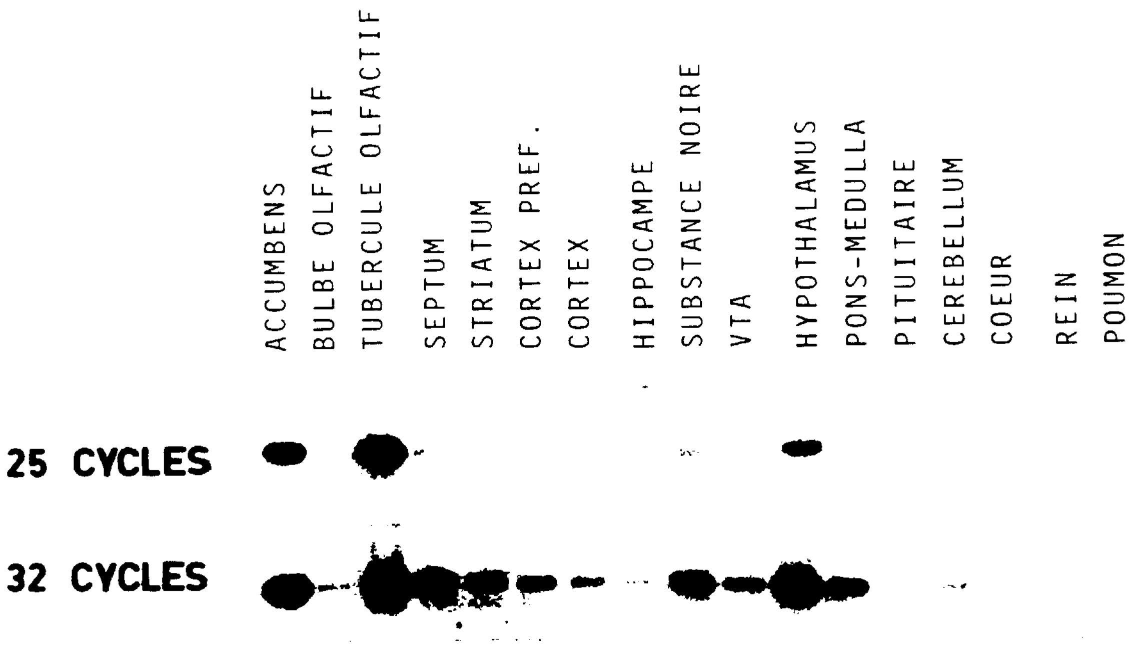
ACCUMBENS
BULBE OLFACTIF
TUBERCULE OLFACTIF
SEPTUM
STRIATUM
CORTEX PREF.
CORTEX
HIPPOCAMPE
SUBSTANCE NOIRE
VTA
HYPOTHALAMUS
PONS-MEDULLA
PITUITAIRE
CEREBELLUM
COEUR
REIN
POUMON
25 CYCLES
32 CYCLES

Figure 8b

Figure 9

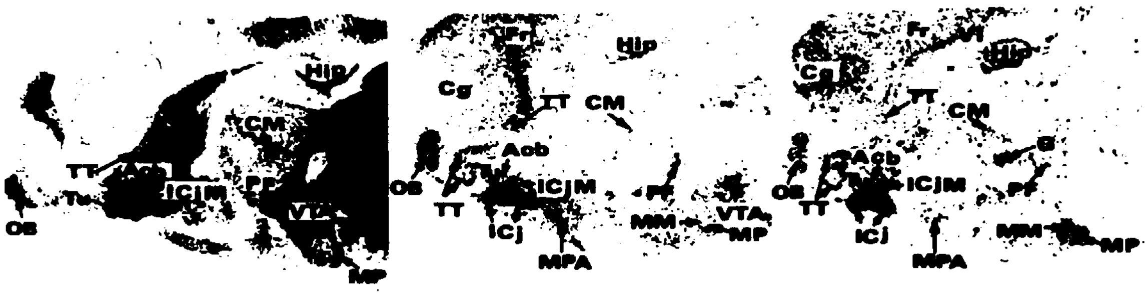


A B C

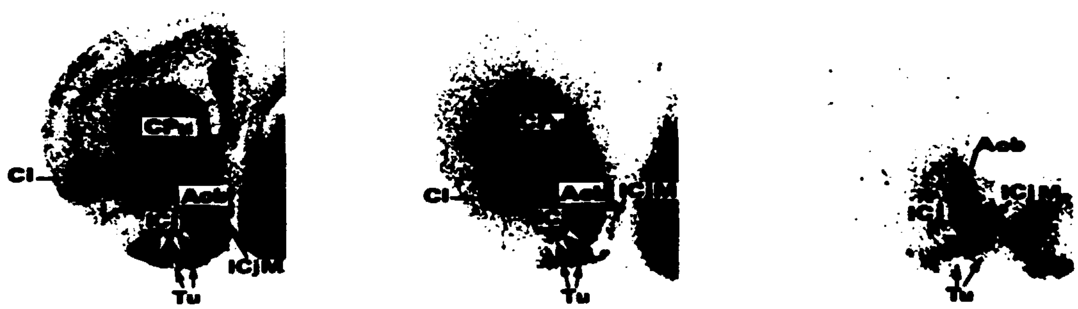


D E F

IODOSULPRIDE-I125 ARNm D-2 ARNm D-3

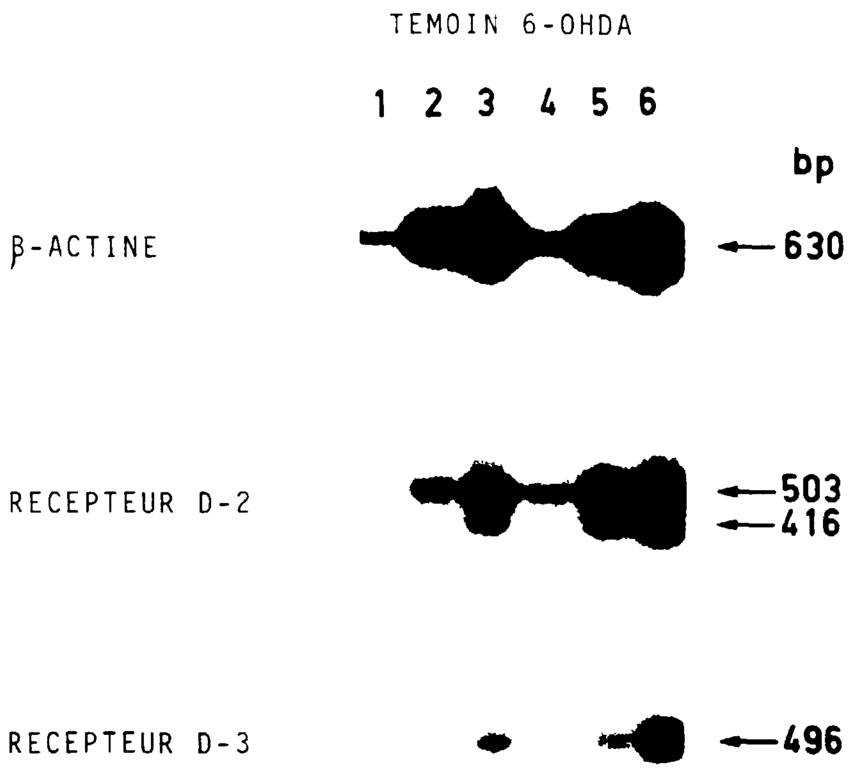
TEMOIN 6-OHDA
1 2 3 4 5 6
bp
β-ACTINE
630
RECEPTEUR D-2
503
416
RECEPTEUR D-3
496

Figure 10

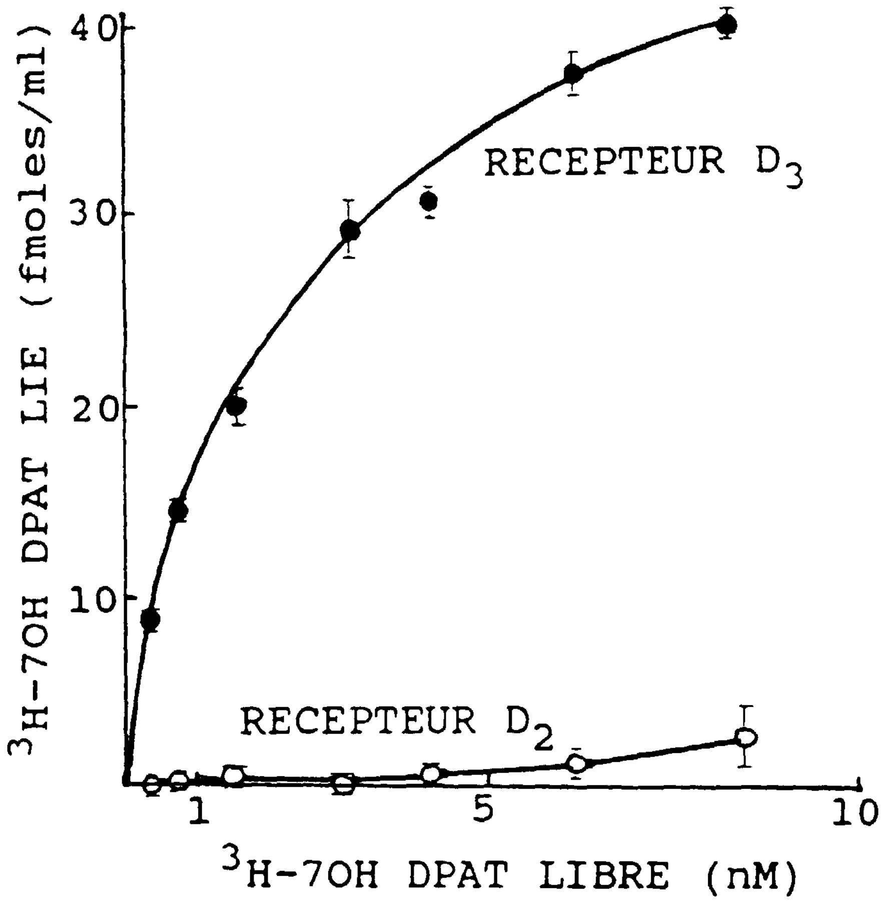

40
30
20
10
RECEPTEUR $D_3$
RECEPTEUR $D_2$
1
5
10
$^3$H-7OH DPAT LIE (fmoles/ml)
$^3$H-7OH DPAT LIBRE (nM)


Figure 11